# EUROPEAN PATENT SPECIFICATION

(11) **EP 3 528 805 B1**
(45) Date of publication and mention of the grant of the patent: **06.12.2023**
(21) Application number: 17861447.5
(22) Date of filing: 21.10.2017
(51) Int. Cl.: C09B 23/01, A61K 31/337, A01N 43/02, A61K 31/35, A61P 35/00, A61P 35/04, C07D 209/10, C07D 405/14, C07D 493/18, A61K 31/22, A61K 31/282, A61K 31/366, A61K 31/40, A61K 31/704, A61K 31/7068, A61K 31/505, A61K 31/555, A61K 33/243, A61K 31/404, A61K 31/4045, A61K 31/47

(54) **SIMVASTATIN AND CHEMOTHERAPEUTIC CONJUGATES COMPRISING A HEPTAMETHINE CARBOCYANINE DYE FOR RESENSITISING HUMAN TUMORS TO HORMONAL ANTAGONISTS AND CHEMOTHERAPY**
SIMVASTATIN UND CHEMOTHERAPEUTISCHE KONJUGATE MIT EINEM HEPTAMETHINCARBOCYANINFARBSTOFF ZUR RESENSIBILISIERUNG VON MENSCHLICHEN TUMOREN GEGEN HORMONALE ANTAGONISTEN UND CHEMOTHERAPIE
SIMVASTATINE ET CONJUGUÉS CHIMIOTHÉRAPEUTIQUES AVEC COLORANT CARBOCYANINE HEPTAMÉTHINE RESENSIBILISANT DES TUMEURS HUMAINES VIS À VIS DES ANTAGONISTES HORMONAUX ET DE LA CHIMIOTHÉRAPIE

(30) Priority: 21.10.2016 US 201662410960 P
(43) Date of publication of application: 28.08.2019
(73) Proprietor: Cedars-Sinai Medical Center, Los Angeles, CA 90048 (US)
(72) Inventor: CHUNG, Leland W. K., Beverly Hills, California 902101 (US); MRDENOVIC, Stefan, 31000 Osijek (HR); ZHANG, Yi, Los Angeles, California 90064 (US); CHU, Gina Chia-Yi, Los Angeles, California 90034 (US); WANG, Ruoxiang, Los Angeles, California 90048 (US); CHANG, Frank N., Dresher, Pennsylvania 19025 (US); TUSZYNSKI, George Paul, Pittsgrove, New Jersey 08318 (US)
(74) Representative: Ellis, Michael James
(86) International application number: PCT/US2017/057765
(87) International publication number: WO 2018/075996

(56) References cited:
- WO-A1-2013/052776
- WO-A1-2013/052776
- WO-A1-2014/086942
- WO-A1-2015/188934
- WO-A1-2016/106324
- WO-A1-2016/106324
- CN-A- 104 312 194
- US-A1- 2012 219 570
- US-A1- 2013 039 854
- US-A1- 2014 248 213
- JASON BOYANG WU ET AL: "Near-infrared fluorescence heptamethine carbocyanine dyes mediate imaging and targeted drug delivery for human brain tumor", BIOMATERIALS, vol. 67, 1 October 2015 (2015-10-01), pages 1-10, XP055480650, AMSTERDAM, NL ISSN: 0142-9612, DOI: 10.1016/j.biomaterials.2015.07.028
- Jason Boyang Wu ET AL: "Supplementary Data Near-infrared fluorescence heptamethine carbocyanine dyes mediate imaging and targeted drug delivery for human brain tumor, Appendix A. Supplementary data", , 1 October 2015 (2015-10-01), XP055695346, Retrieved from the Internet: URL:https://ars.els-cdn.com/content/image/ 1-s2.0-S0142961215006109-mmc1.pdf [retrieved on 2020-05-14]
- YANG GUAN ET AL: "Improving Therapeutic Potential of Farnesylthiosalicylic Acid: Tumor Specific Delivery via Conjugation with Heptamethine Cyanine Dye", MOLECULAR PHARMACEUTICS, vol. 14, no. 1, 21 March 2016 (2016-03-21) , pages 1-13, XP055451586, US ISSN: 1543-8384, DOI: 10.1021/acs.molpharmaceut.5b00906
- KI-EUN HWANG ET AL: "Effect of simvastatin on the resistance to EGFR tyrosine kinase inhibitors in a non-small cell lung cancer with the T790M mutation of EGFR", EXPERIMENTAL CELL RESEARCH, vol. 323, no. 2, 1 May 2014 (2014-05-01), pages 288-296, XP055696397, AMSTERDAM, NL ISSN: 0014-4827, DOI: 10.1016/j.yexcr.2014.02.026

## Description

### FIELD OF THE INVENTION

This application relates to dye-therapeutic moiety conjugates for imaging, targeting, and treating cancerous cells and/or tumors.

### CROSS-REFERENCE TO RELATED APPLICATIONS

This application includes a claim of priority to U.S. provisional patent application No. 62/410960, filed October 21, 2016.

### BACKGROUND

The following description includes information that may be useful in understanding the present invention. It is not an admission that any of the information provided herein is prior art or relevant to the presently claimed invention, or that any publication specifically or implicitly referenced is prior art.

Currently there is a lack of effective treatment agents for cancers and cancer metastases, particularly drug resistant cancers. Therefore there is a need in the art for additional cancer therapies as well as new imaging agents to see live tumor cells and tumor tissues in patients.

Simvastatin (SM), a statin drug that has been shown to lower serum cholesterol level in man though host liver, is a potent drug for the prevention of cardiovascular diseases. As described herein, we discovered that SM can be safely delivered to cancer cells through conjugation with near-infrared (NIR) organic dyes, e.g., a heptamethine carbocyanine, to form a conjugate that can target cancer cells through a series of organic anion transporting peptides (OATPs) carriers. We found NIR-SM conjugates to be a potent tumor-acting drug. For example, as described herein, we synthesized several unique dye-therapeutic moiety conjugates (dye-THM conjugates) through conjugation of therapeutic agents, e.g., chemotherapeutic agents, to dyes, e.g., NIR organic dyes. All of the synthesized dye-therapeutic moiety conjugates were found to have unique anti-cancer capability while avoiding host toxicity. The dye-therapeutic moiety conjugates of the invention and the various embodiments described herein may be used in a variety of applications, including without limitation, as enhancers for existing therapeutics, as direct tumor targeting agents, as imaging agents to visualize live cells and/or tumors, for the treatment of tumors, for the treatment of tumors and their metastases, for the treatment of tumors and their metastases that are therapeutic resistant, as direct agents to synergize with other agents, as agents for visualizing live tumor cells and tissues, to assist surgeons during operations, and as agents to image tumor and tumor metastases. WO-A1-2016/106324 discloses dye-conjugates which may comprise a variety of radionuclides for radionuclear imaging, including, among others, F18.

### SUMMARY

The following embodiments and aspects thereof are described and illustrated in conjunction with compositions and methods which are meant to be exemplary and illustrative, not limiting in scope. This application relates to dye-therapeutic moiety conjugates for imaging, targeting, and treating cancerous cells and/or tumors as described herein.

### BRIEF DESCRIPTION OF THE DRAWINGS

Exemplary embodiments are illustrated in referenced figures. It is intended that the embodiments and figures disclosed herein are to be considered illustrative rather than restrictive.

The figures disclosed herein are of illustrative embodiments. They do not illustrate all embodiments. Other embodiments may be used in addition or instead. Details that may be apparent or unnecessary may be omitted to save space or for more effective illustration. Some embodiments may be practiced with additional components or steps and/or without all of the components or steps that are illustrated. The numerical values shown on the Figures are for illustrative purposes and should not be construed to be limiting the scope of the invention in any way.

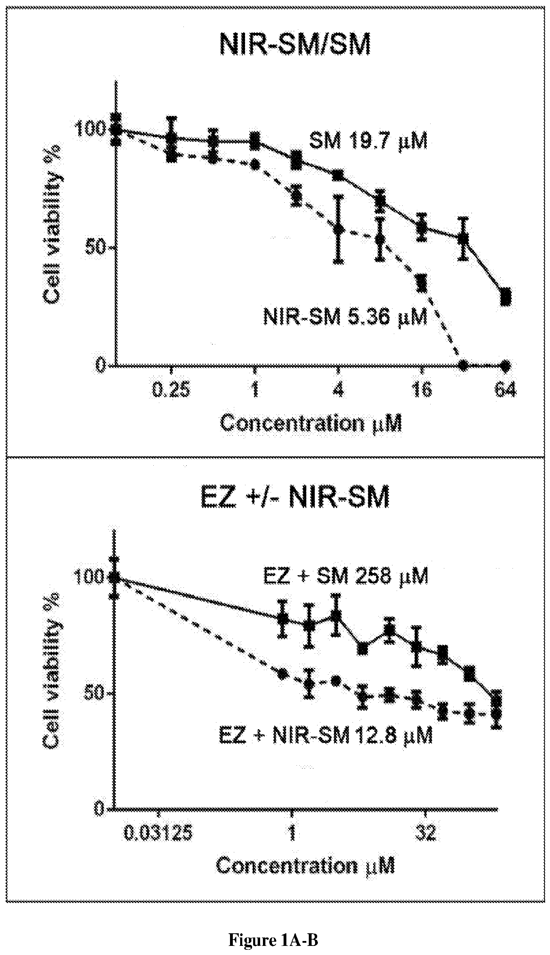

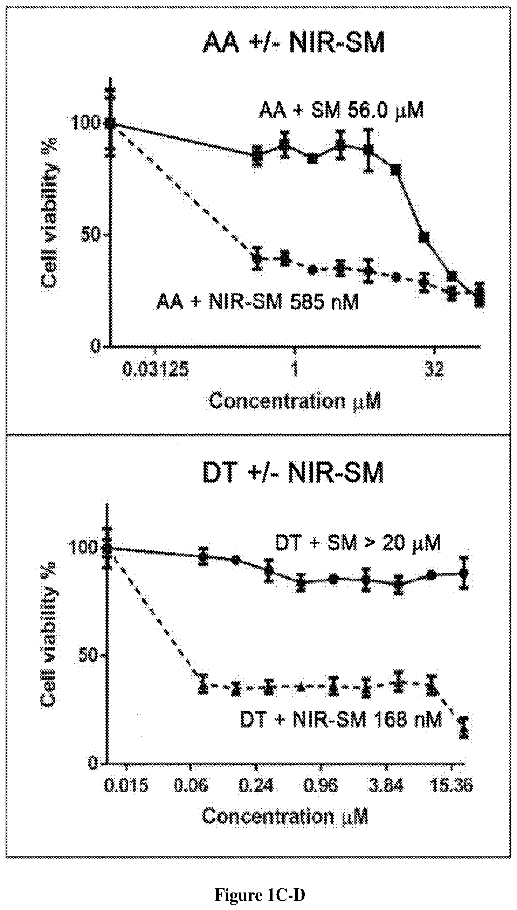

**Figure** 1 depicts in accordance with various embodiments of the invention, NIR-SM but not SM sensitizes AA, EZ and DT induced cytotoxicity of 3D CTCs spheroid culture.

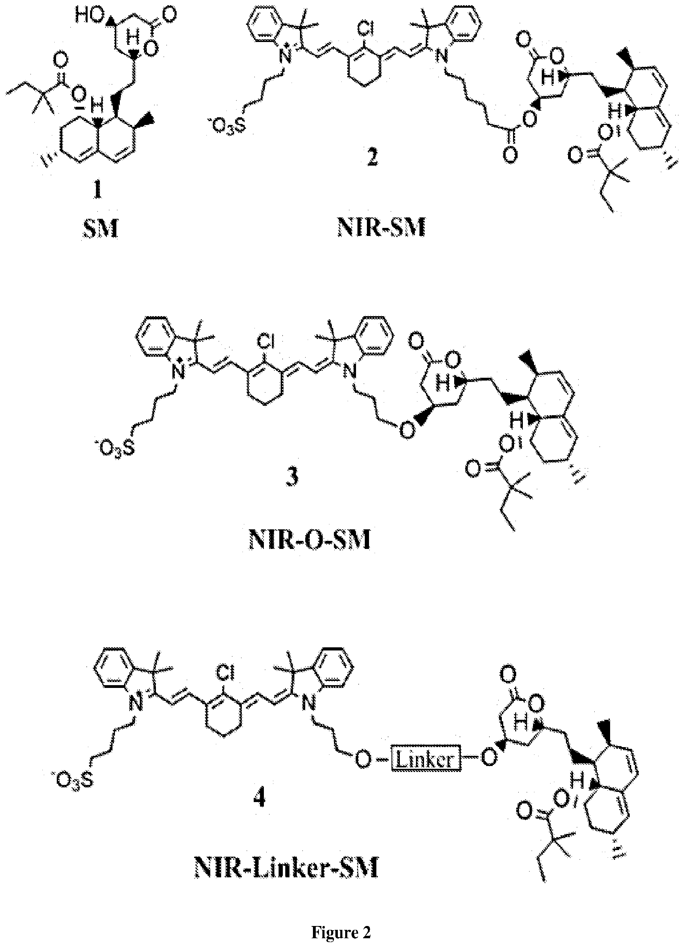

**Figure 2** depicts in accordance with various embodiments of the invention, chemical structure of SM and NIR-SM conjugates.

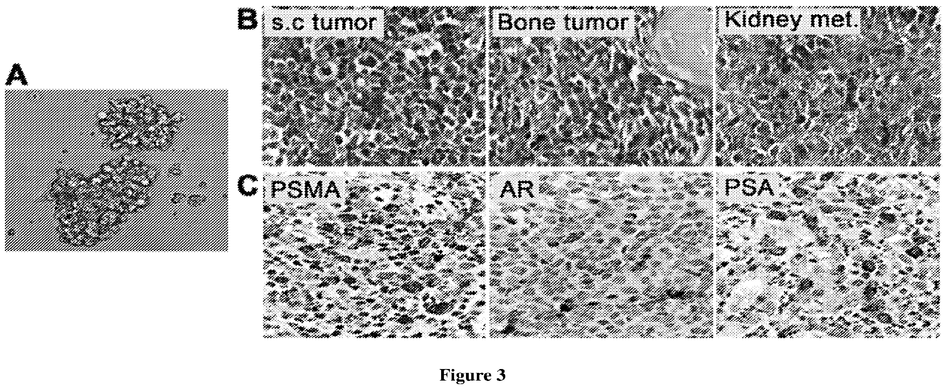

**Figure 3** depicts in accordance with various embodiments of the invention, morphology of 3D CTC spheroids (A), metastasis to bone and kidney (B), and immunohistochemistry of a PC CDX model showing positive staining of PSMA, AR and PSA (C).

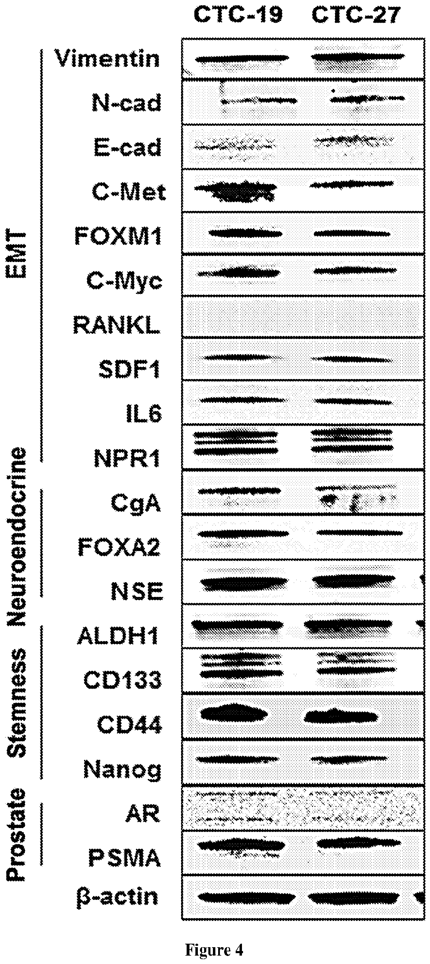

**Figure 4** depicts in accordance with various embodiments of the invention, two representative *ex vivo* expanded CTC spheroids showed the expression of EMT, neuroendocrine, sternness and prostate cancer biomarkers.

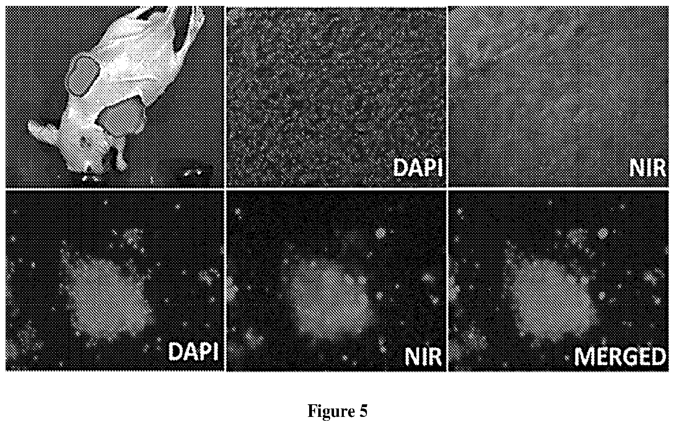

**Figure 5** depicts in accordance with various embodiments of the invention, NIR-SM was found to be uptake and retained in 2D monolayer of PC3 cells (top right), 3D CTCs spheroids (bottom), and retained exclusively by PC-3 tumors implanted bilaterally at subcutaneous space of a mouse (top left).

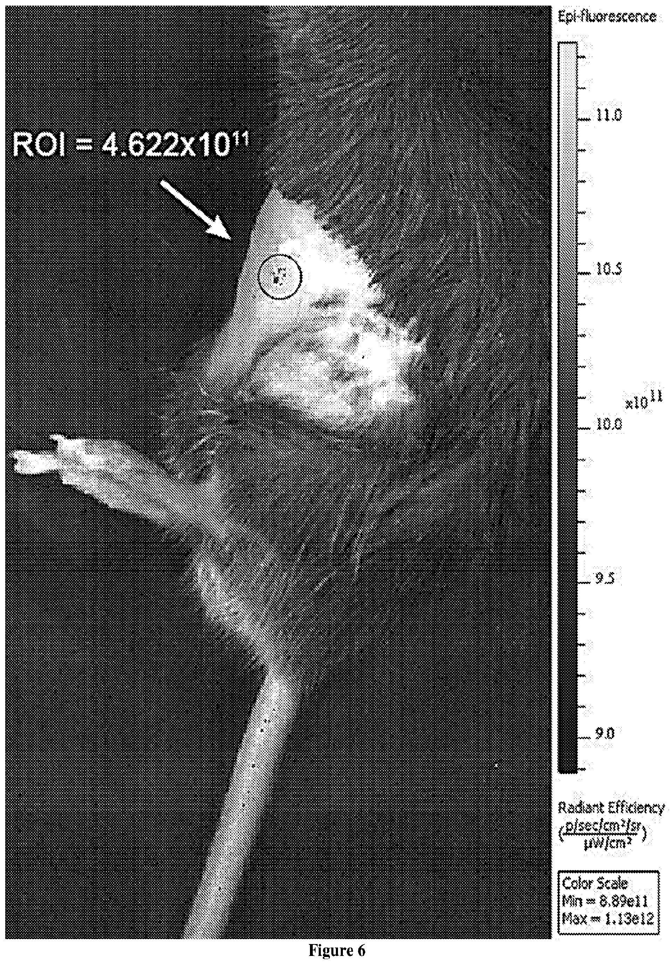

**Figure 6** depicts in accordance with various embodiments of the invention, NIR tumor imaging shows that NIR-SM targets specifically PDAC tumors in KPC mice (imaged 3 days after intraperitoneal (i.p.) administration of the NIR-SM conjugate). The PDAC tumor at the time of imaging was estimated to be 1.1 mm in diameter (arrow).

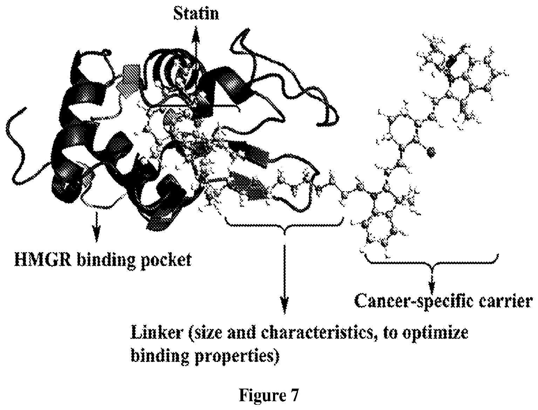

**Figure 7** depicts in accordance with various embodiments of the invention, conceptual design by docking NIR-Linker-SM to HMGR binding pocket.

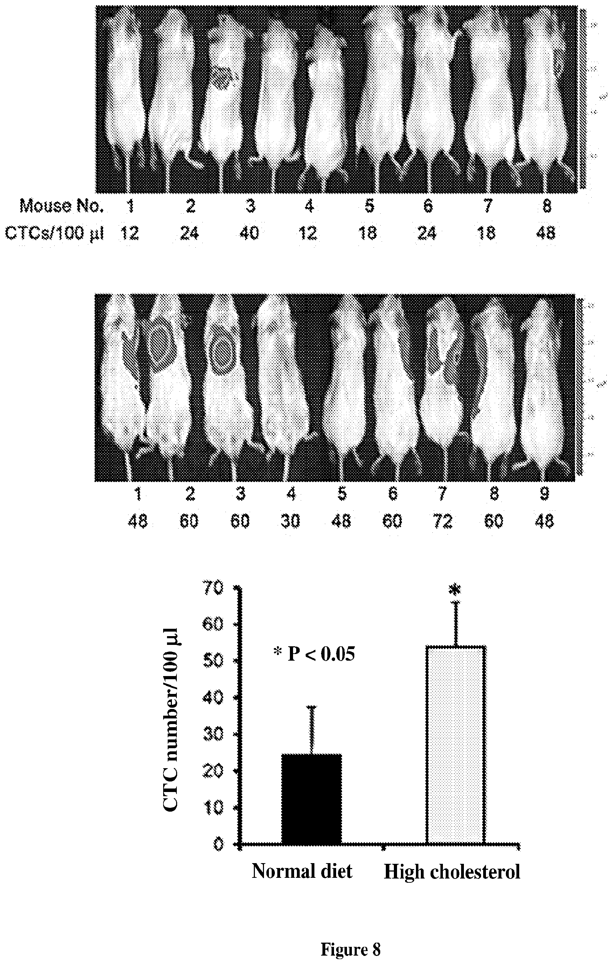

**Figure 8** depicts in accordance with various embodiments of the invention, mice (N=9 per group) were subjected to i.c injection of LN^{RANKL/Luc/RFP} cells. Mice were imaged every two weeks and their CTC counts were obtained on the cytospin slides. Note higher number of metastasis, detected by bioluminescence, accompanied by higher number of CTCs in high cholesterol diet fed (right group) than normal diet fed mice (left group) at 12 weeks.

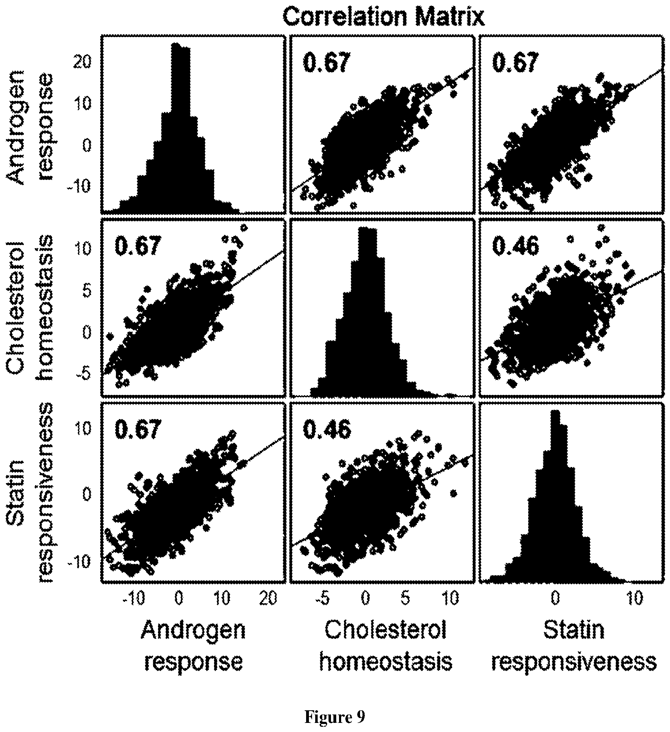

**Figure 9** depicts in accordance with various embodiments of the invention, correlation between the signatures of androgen response, cholesterol homeostasis, and statin responsiveness. The values in red on the plots represent Spearman's correlation coefficient. All three signatures show significant positive correlation (P<0.01) in the PC cohort.

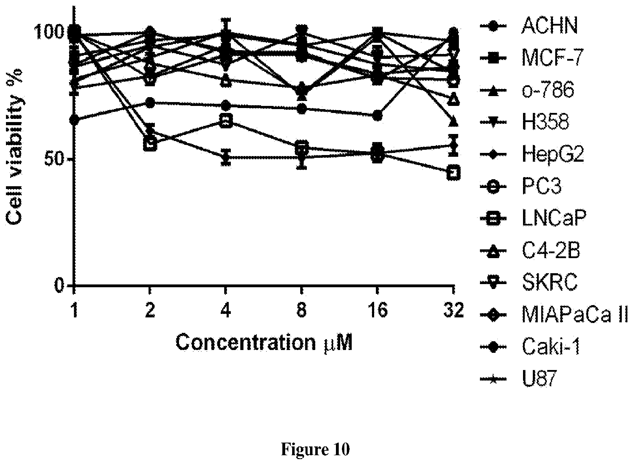

**Figure 10** depicts in accordance with various embodiments of the invention, a prototype of near-infrared heptamethine carbocyanine dye, NIR-783, does not exhibit significant cell inhibition effect on renal (ACHN, o-786, SKRC, Caki-1), breast (MCF-7), lung (H358), liver (HepG2), prostate (PC3, LNCaP, C4-2B), pancreatic (MIAPaCa II) and glioblastoma (U87) cancer cells *in vitro.*

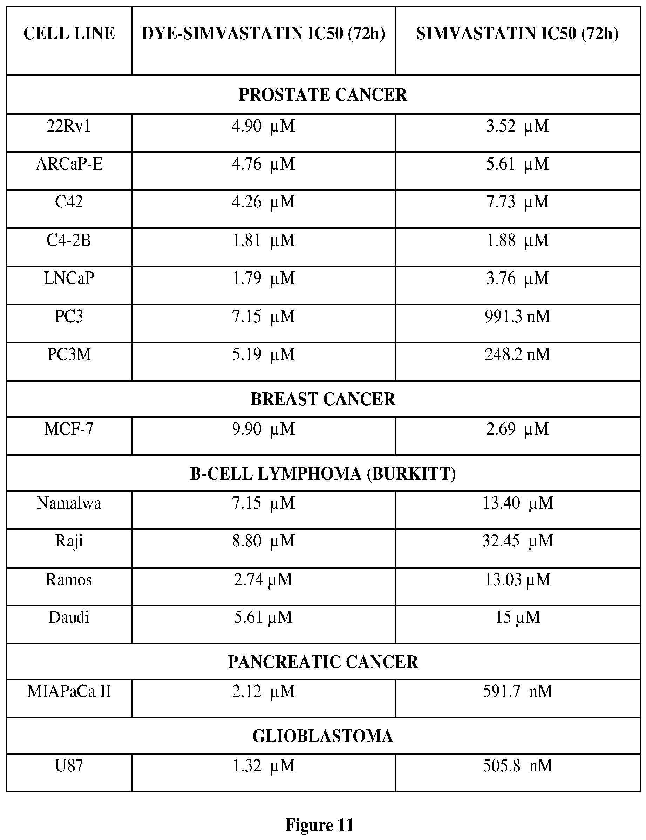

**Figure 11** depicts in accordance with various embodiments of the invention, comparison of IC50s of dye-simvastatin and simvastatin in cultured prostate, breast, B-cell lymphoma, pancreatic and glioblastoma cancer cell lines.

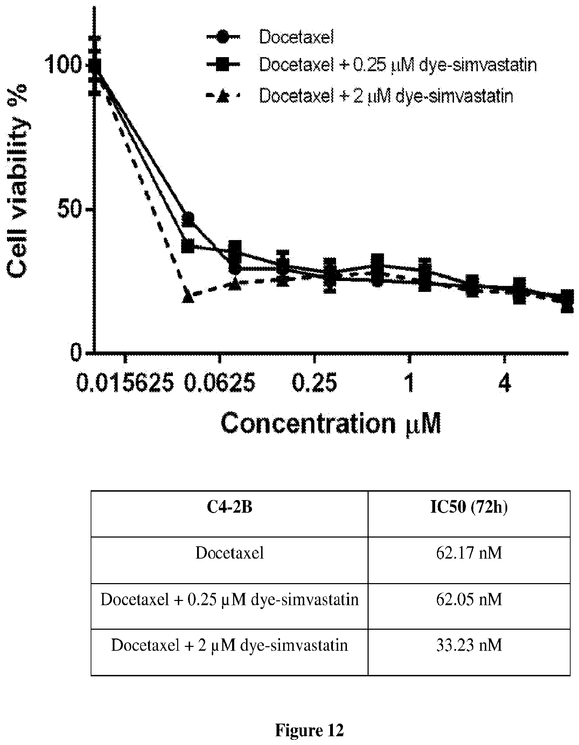

**Figure 12** depicts in accordance with various embodiments of the invention, dye-simvastatin conjugate at 2 uM synergizes the growth inhibitory effects of docetaxel on castration-resistant prostate cancer cell line, C4-2B cells in vitro.

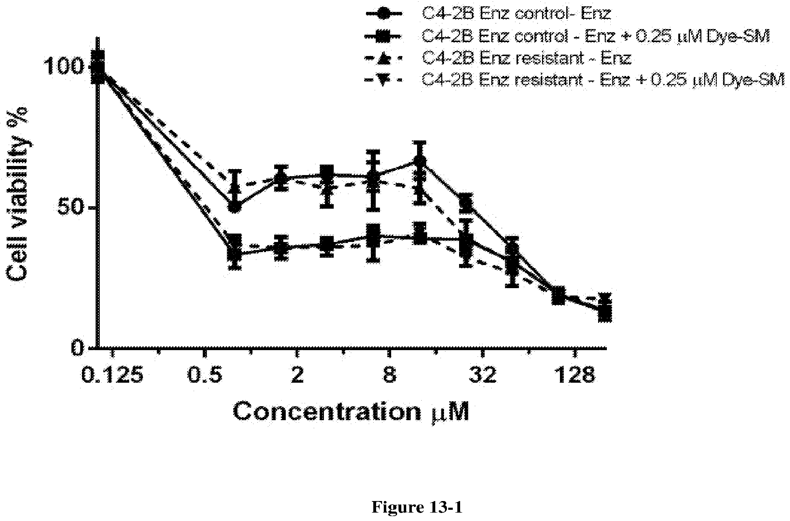

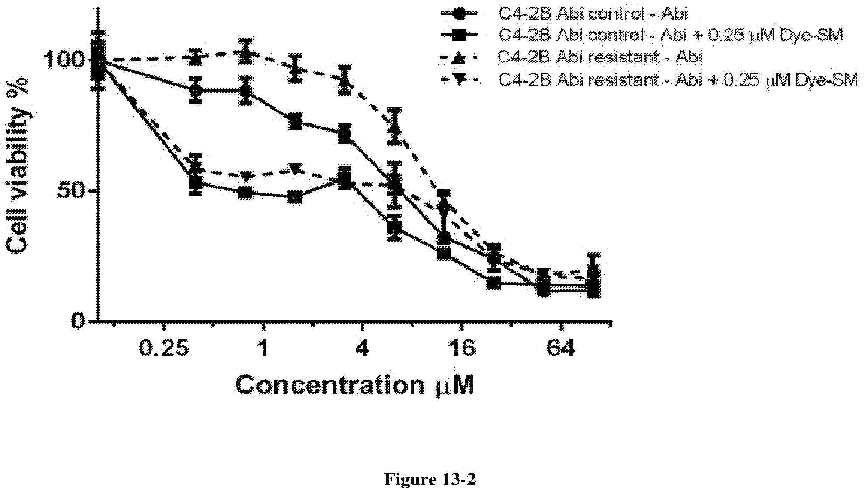

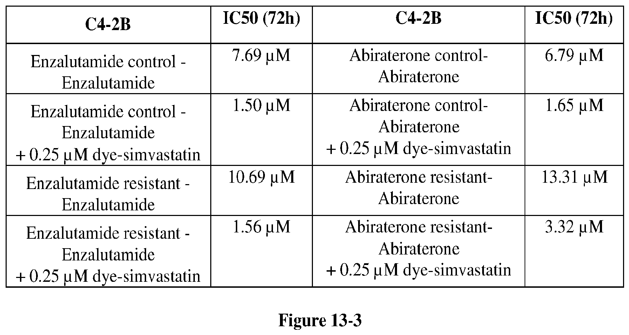

**Figure 13** depicts in accordance with various embodiments of the invention, dye-simvastatin conjugate synergizes the growth inhibitory effects of anti-androgens, enzalutamide (Enz) and abiraterone acetate (Abi), on the growth of castration-resistant C4-2B cells in vitro.

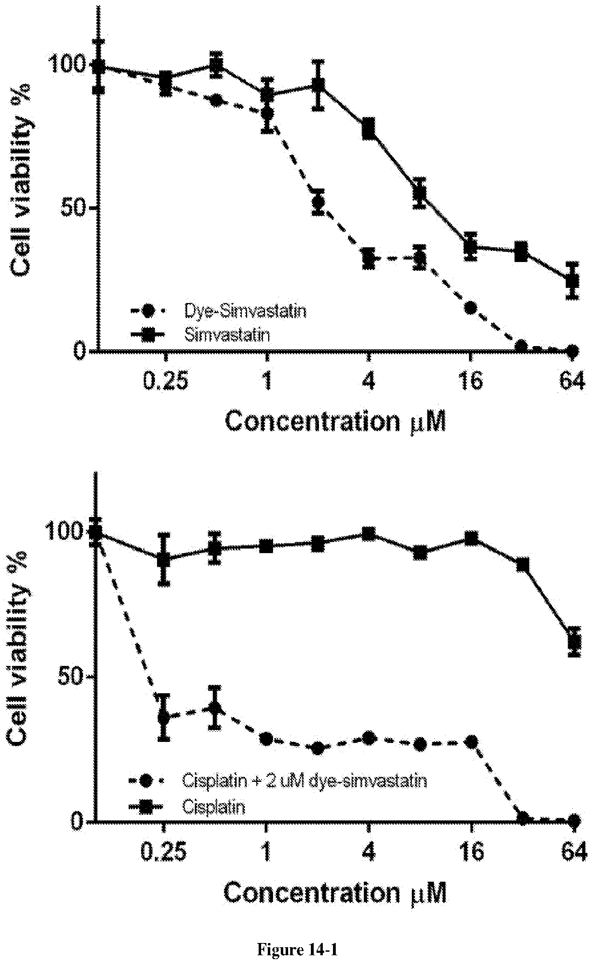

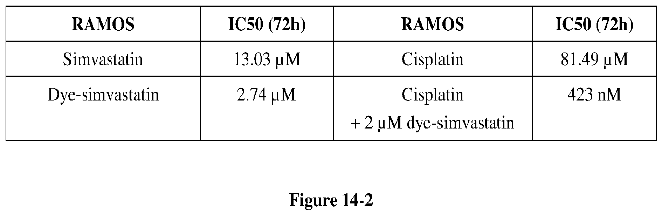

**Figure 14** depicts in accordance with various embodiments of the invention, dye-simvastatin conjugate, a cholesterol lowering agent, when delivered directly to cancer cells (Ramos, a B-cell lymphoma), cultured as 3-D spheroids, was about 5-fold more potent than the unconjugated simvastatin, and greatly synergized cisplatin cytotoxicity by over 190-fold.

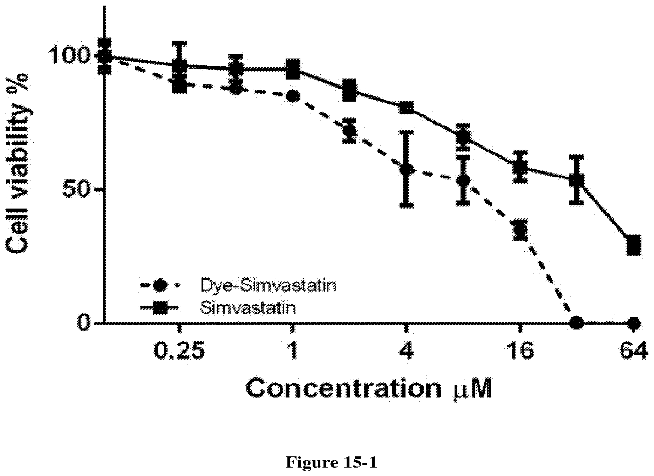

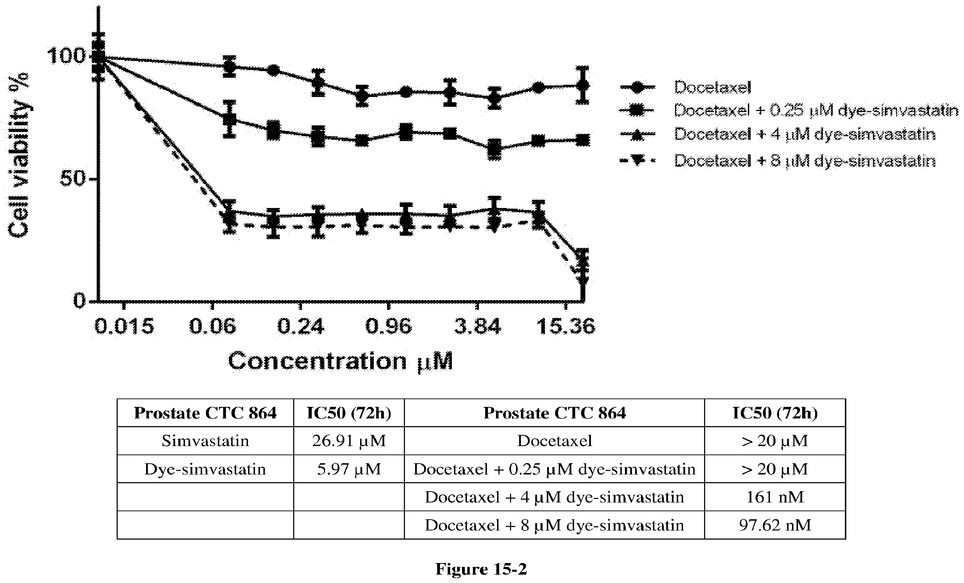

**Figure 15** depicts in accordance with various embodiments of the invention, dye-simvastatin was more effective than unconjugated simvastatin and can greatly enhanced docetaxel cytotoxicity against the growth of prostate cancer CTCs grown as 3-D spheroids in a concentration-dependent manner.

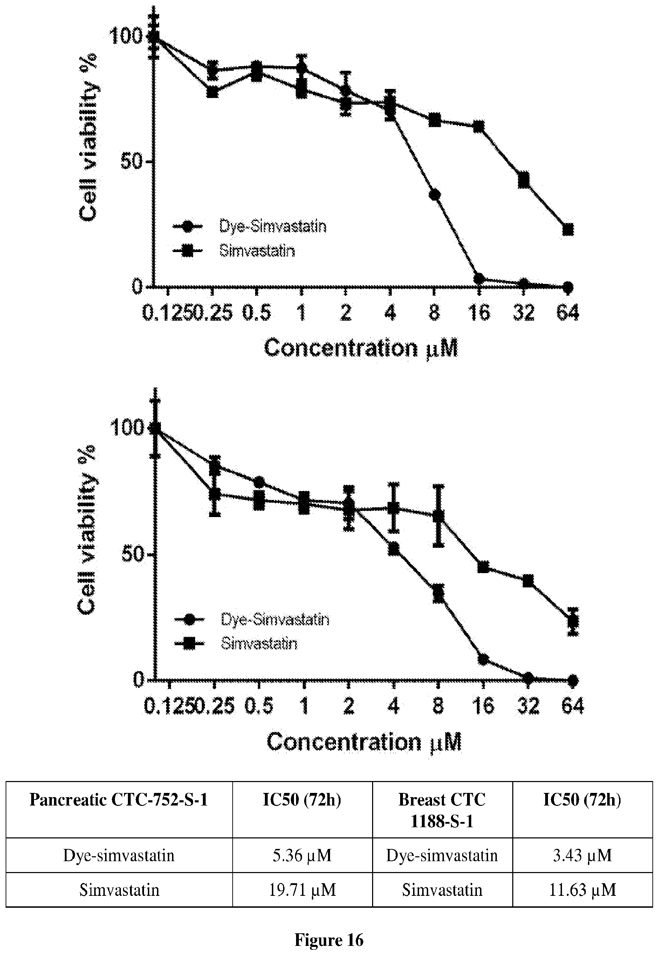

**Figure 16** depicts in accordance with various embodiments of the invention, dye-simvastatin was more effective than unconjugated simvastatin in inhibition of pancreatic and breast cancer CTCs grown as 3-D spheroids.

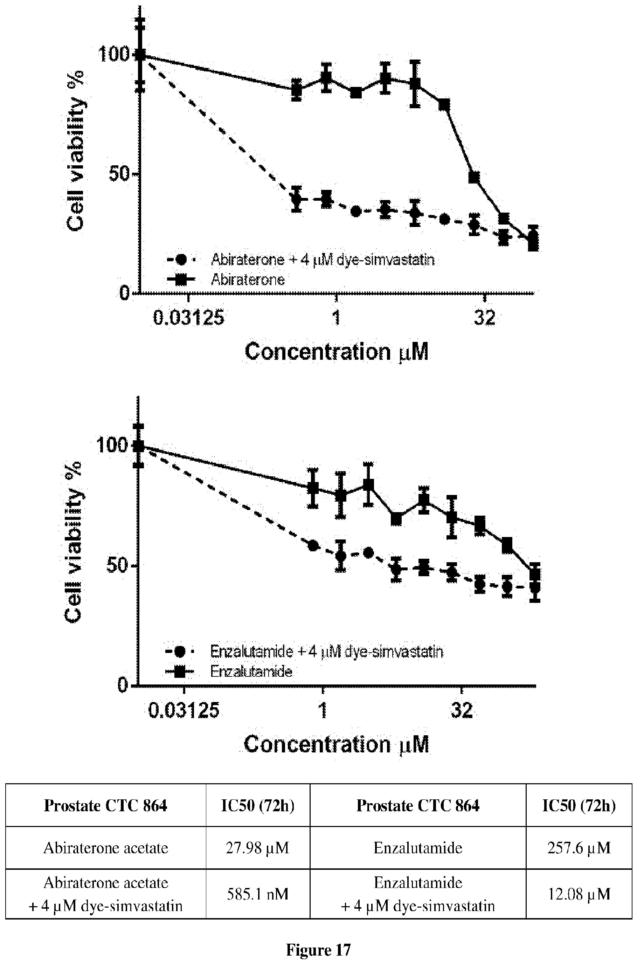

**Figure 17** depicts in accordance with various embodiments of the invention, dye-simvastatin is more effective than unconjugated simvastatin in enhancing abiraterone acetate and enzalutamide-induced cytotoxicity against the growth of prostate cancer CTCs grown as 3-D spheroids.

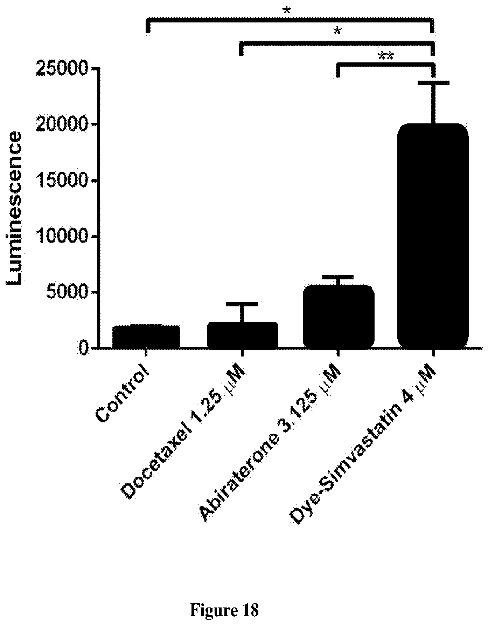

**Figure 18** depicts in accordance with various embodiments of the invention, dye-simvastatin causes better cytotoxicity, as measured by apoptosis assay using a caspase 3/7 assay, than docetaxel and abiraterone acetate, in inhibiting prostate cancer CTCs growth as 3D spheroids. Prostate circulating tumor cels CTC 864 were exposed to docetaxel (1.25 µM), abiraterone acetate (3.125 µM) or dye-simvastatin (4 µM) for 24 hours. Rate of apoptosis was measured using a caspase 3/7 assay. Dye-simvastatin had significantlly higher rate of apoptosis compared to control, docetaxel (*, p < 0.01) and abiraterone acetate (**, p < 0.05).

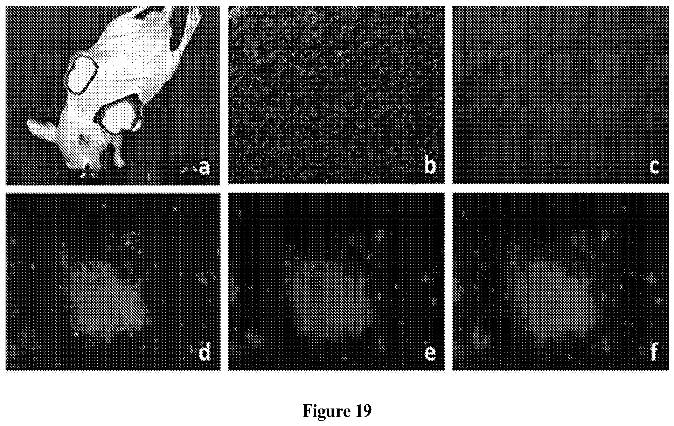

**Figure 19** depicts in accordance with various embodiments of the invention, dye-simvastatin shows selective acumulation in a prostate cancer PC3 tumor xenograft assesed using IVIS Lumina XR Imaging System (a). Evidence of dye-simvastatin accumulation seen in PC3 tumor sections using infrared microscopy on fresh frozen sections (b-DAPI, c-Cy7) and patient derived circulating tumor cells CTC 864 (d-DAPI, e-Cy7, f-merged).

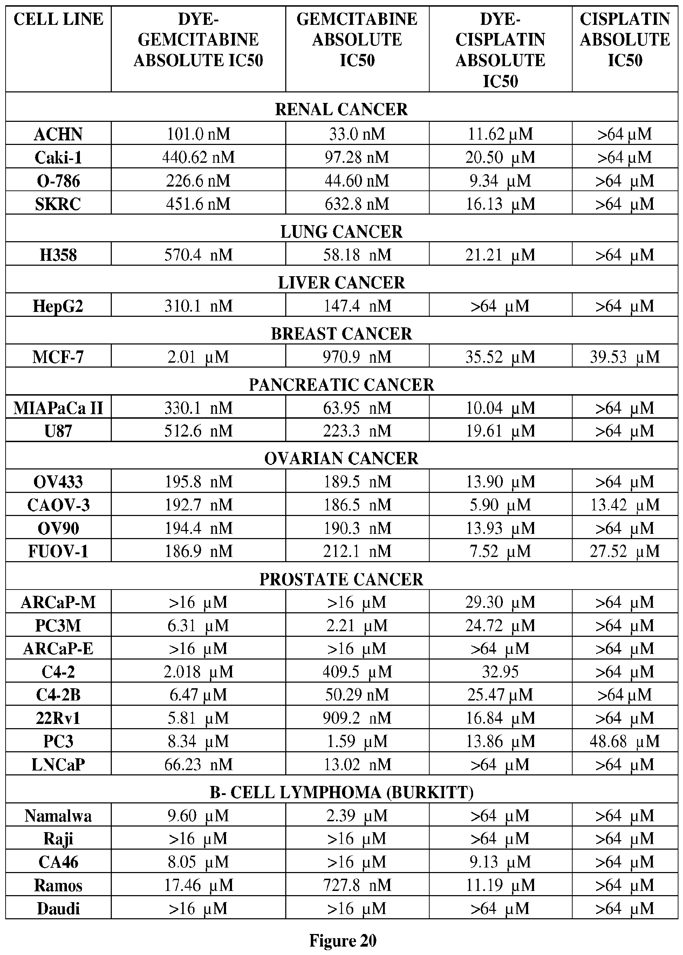

**Figure 20** depicts in accordance with various embodiments of the invention, dye-gemcitabine amide conjugate exhibits similar IC50 values as gemcitabine. Dye-cisplatin amide conjugate exhibits significantly lower IC50 values and overcoming of cisplatin resistance in a number of cell lines tested.

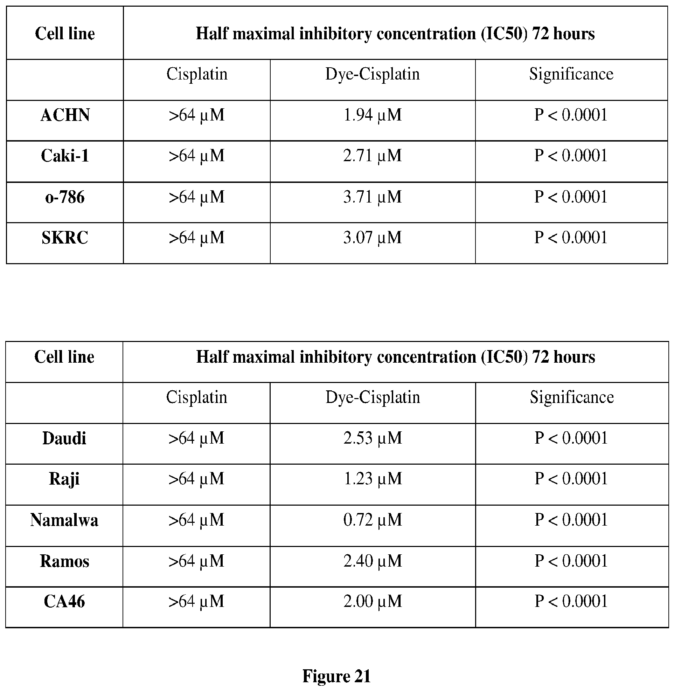

**Figure 21** depicts in accordance with various embodiments of the invention, dye-cisplatin conjugate with ester bond exhibits even more potent antitumor effect as presented on renal cancer (top panel) and B-cell lymphoma cell lines (bottom panel). Renal cancer cell lines, ACHN, Caki-1, o-786, SKRC and immortalized B-cell lymphoma cell lines, Namalwa, Raji, CA46, Ramos, Daudi are supplemented with RPMI 1640 medium, 10% fetal bovine serum, 1% PenStrep, and placed in a humidified atmosphere containing 5% CO2 at 37° C. 5,000 cells/well renal cancer cells and 10,000 cells/well lymphoma cells are seeded in 96 well plates in 100 microliter medium/per well for 24 h. Cell lines are exposed to exponentially increasing concentrations of either cisplatin or dye-cisplatin ranging from 0.25 µM to 64 µM for 72 h. Renal cancer cells are fixed with 3.7% paraformaldehyde for 15 minutes and are stained with Crystal violet solution (0.5% in 25% methanol) for 30 minutes. After rinsing with water cell layers are dried and decolorized with Sorenson citrate with shaking for 20 minutes. Light absorption of the extracted supernatant is read at 595 nm. Lymphoma cell growth was evaluated using MTT and cells are incubated for 4 hours. 100 microliters of isopropanol is added, and the extracted supernatant fraction is read at absorption maximum of 595 nm.

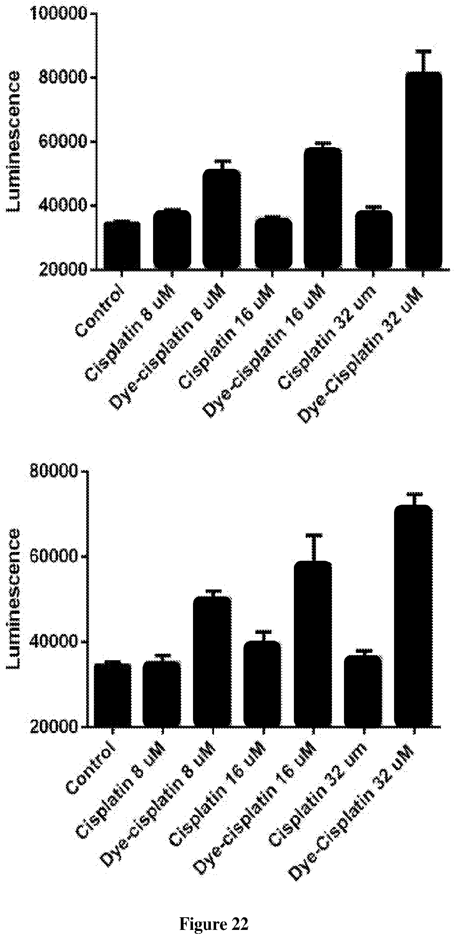

**Figure 22** depicts in accordance with various embodiments of the invention, dye-cisplatin but not cisplatin treatment induces apoptosis in immortalized B-cell lymphoma cell line, Namalwa, and renal cancer cell line, Caki-1, in vitro. Namalwa cells are cultured in RPMI 1640, 10% fetal bovine serum, 1% PenStrep, and placed in a humidified atmosphere containing 5% CO2 at 37° C. 10,000 - 15,000 cells/well are seeded in 96 white wall well plates in 100 microliter medium/per well for 24 h. Cell lines are then exposed to either cisplatin or dye-cisplatin for 24 hours. 100 microliters of Caspase-Glo 3/7 assay is added and luminescence is then read. Caki-1 cells are cultured in RPMI 1640, 10% fetal bovine serum, 1% PenStrep, and placed in a humidified atmosphere containing 5% CO2 at 37° C. 5,000 cells/well are seeded in 96 white wall well plates in 100 microliter medium/per well for 24 h. Cell lines are then exposed to either cisplatin or dye-cisplatin for 24 hours. 100 microliters of Caspase-Glo 3/7 assay is added and luminescence is then read.

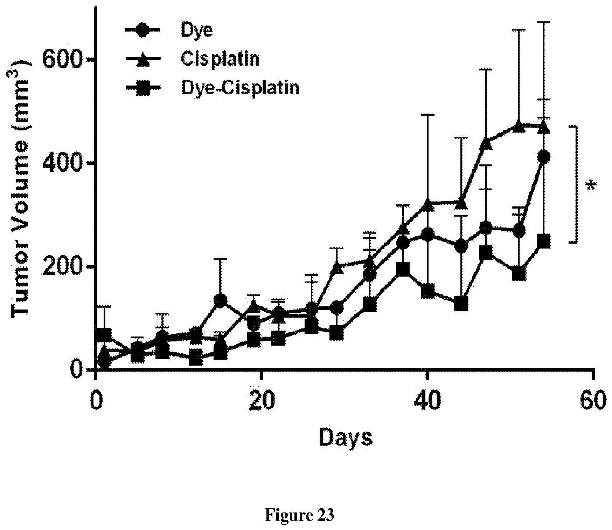

**Figure 23** depicts in accordance with various embodiments of the invention, equal molar concentration of dye-cisplatin is more effective than cisplatin in inhibiting renal cancer cell line (ACHN) xenograft growth in immune-compromised mice: 8 x 106 Caki-1 cells are injected subcutaneously in 4 week old NCR-NU mice. When the tumors grow to 50 mm3, treatment is started with 10 mg/kg NIR-783 dye, 7 mg/kg cisplatin or equimolar dose of dye-cisplatin 3 times a week i.p. Tumor size is measured two times a week. Tumor size is measured using a formula (length × width²) / 2.

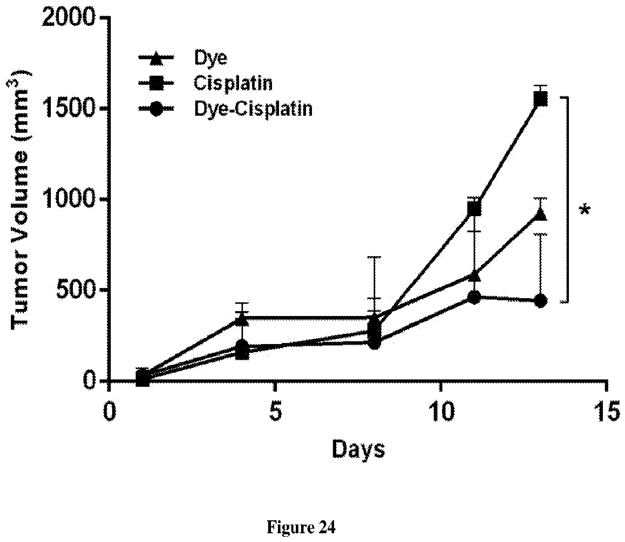

**Figure 24** depicts in accordance with various embodiments of the invention, a syngenic mouse model of mouse renal cancer cell line (Renca) showed dye-cisplatin is more effective than cisplatin in inhibiting tumor growth in immune-intact Balb/c mice: 500 000 Renca cells are injected subcutaneously in 4 week old BALB/c mice. When the tumors grow to ~50 mm3 in size, treatment is started with 10 mg/kg NIR-783 dye, 7 mg/kg cisplatin or equimolar dose of dye-cisplatin 3 times a week i.p. Tumor size is measured two times a week. Tumor size is measured using a formula (length × width²) / 2. Note dye-cisplatin is more effective than cisplatin in inhibiting Renca tumor growth in mice.

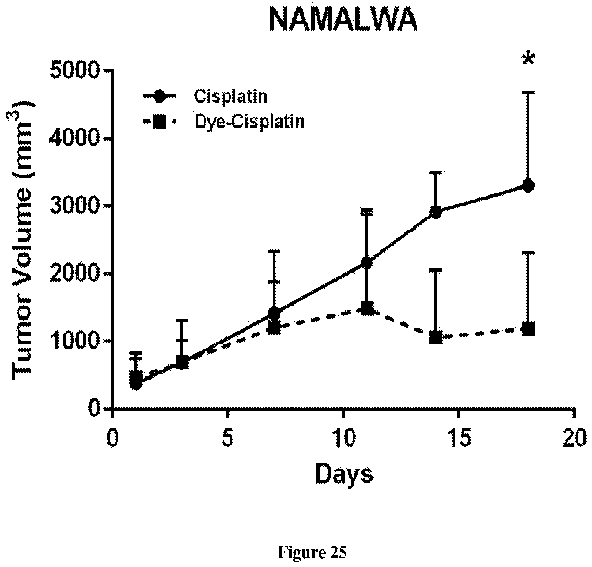

**Figure 25** depicts in accordance with various embodiments of the invention, dye-cisplatin is more effective than cisplatin in inhibiting the growth of B-cell lymphoma (Namalwa) tumor growth in mice: 10⁷ Namalwa cells are injected subcutaneously in 4 week old NCR-NU mice. When the tumors grow to ~50 mm3 in size, treatment is started with 7 mg/kg cisplatin or equimolar dose of dye-cisplatin 3 times a week i.p. Tumor size is measured two times a week. Tumor size is measured using a formula (length × width²) / 2.

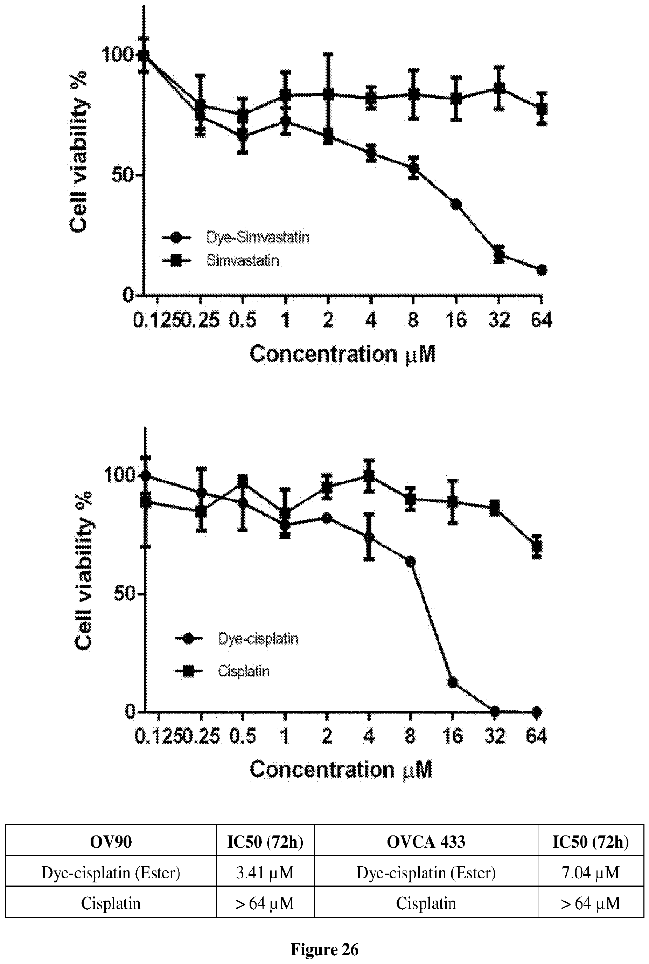

**Figure 26** depicts in accordance with various embodiments of the invention, the growth of ovarian tumor cells, OV90 and OVCA433, in vitro is more sensitive toward the growth inhibitory effects of dye-simvastatin and dye-cisplatin than the unconjugated simvastatin and cisplatin.

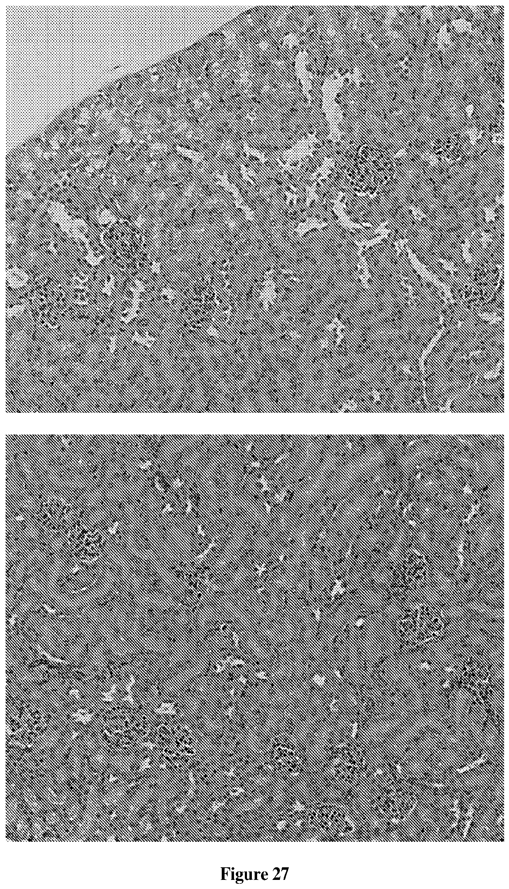

**Figure 27** depicts in accordance with various embodiments of the invention, formalin-fixed kidney sections are stained with hematoxylin and esosin and scored using a semiquantitative scale designed to assess AKI-associated tubular injury (tubular epithelial cell loss, necrosis, tubular epithelial simplification, intratubular debris, and casts). NIR-dye-cisplatin preserves the integrity and normal morphology of mouse kidneys (right panel), and by contrast, cisplatin is observed to induce marked kidney damage as assessed by the histopathology of cisplatin-treated mice (left panel).

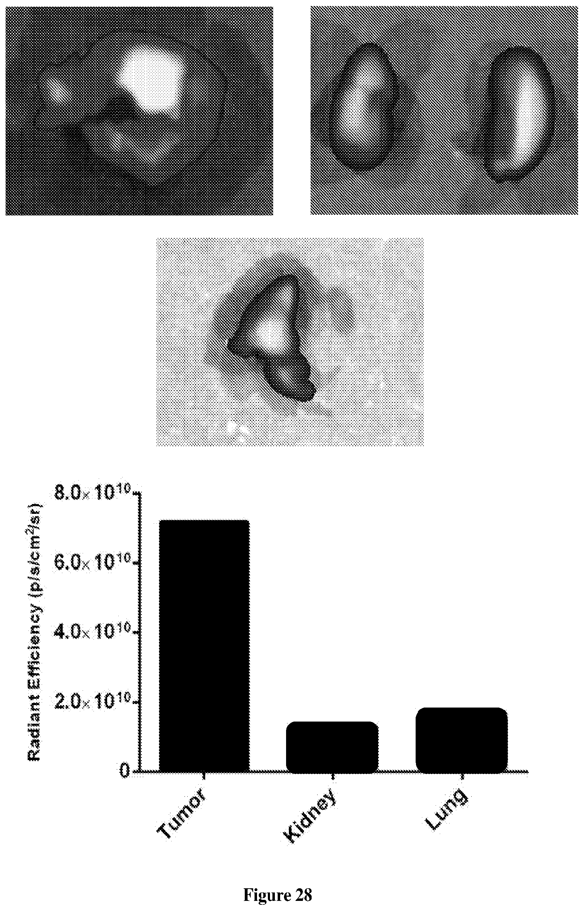

**Figure 28** depicts in accordance with various embodiments of the invention, B-cell lymphoma cell line (Namalwa) subcutaneus tumor xenografts in NCR-NU mice were extracted after the mice were sacrificed. NIR-dye-cisplatin uptake in tumors were imaged by a near-infrared fluorescence (NIRF) microscopy and IVIS Lumina XR Imaging System. NIR-dye-cisplatin was found to accumulate 7 to 15 times more in cell line-derived xenografts than mouse kidneys (p<0.0001) and lungs (p<0.0001).

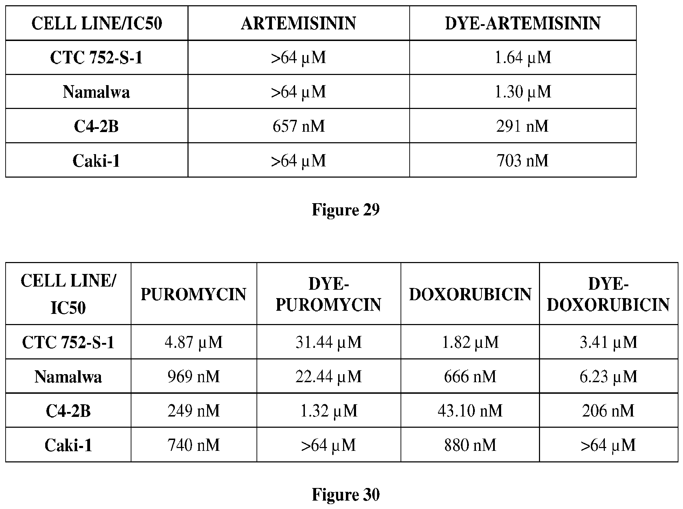

**Figure 29** depicts in accordance with various embodiments of the invention, dye-artemisinin conjugate greatly enhances the tumor growth inhibitory effects of artemisinin in a number of tumor types as represented by their IC50s in 3D cultured circulating tumor cells, CTCs grown as tumor spheroids (CTC 752-S-1), B-cell lymphoma (Namalwa), castration-resistant prostate cancer (C4-2B), and renal cancer (Caki-1).
**Figure 30** depicts in accordance with various embodiments of the invention, NIR dye-artemisinin but not NIR dye-puromycin and NIR dye-doxorubicin showed, greatly enhanced cytoxixic effects than the parental drugs against the growth of CTCs (C752-S-1), B-Cell Lymphoma (Namalwa), prostate (C4-2B), and Renal (Caki-1) cancer cells *in vitro.*

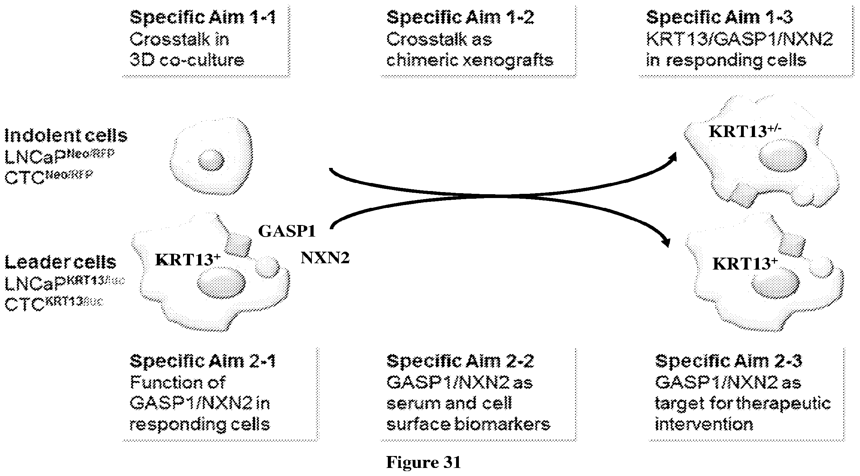

**Figure 31** depicts a schematic overview of the interaction between indolent prostate cancer cells and aggressive leader cells, which express three molecular markers: keratin 13 (KRT13), G protein coupled receptor associated sorting protein 1 (GASP-1), and neuroredoxin 2 (NXN2). LNCaP or CTC may be tagged genetically with a red fluorescent protein (RFP). Without wishing to be bound by theory, it is believed that aggressive leader cells can confer tumorigenic and metastatic potential to the indolent prostate cancer cells, by transferring the three molecular markers from the aggressive to the indolent prostate cancer cells.

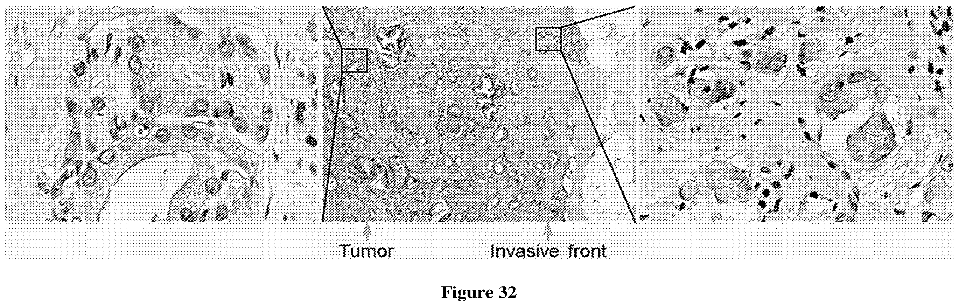

**Figure 32** depicts the expression of KRT13, as detected by immunohistochemistry (IHC), in the invasive front of the human prostate cancer tissues.

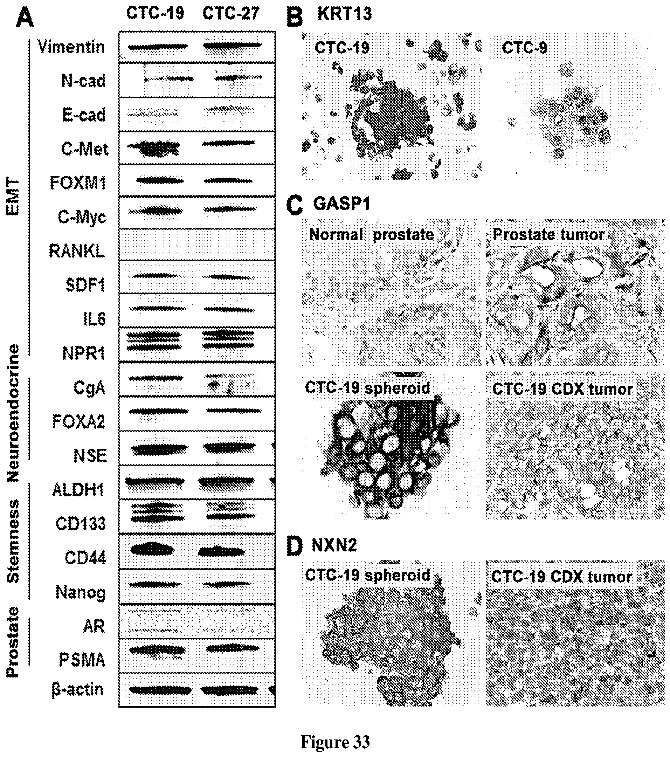

**Figure 33** depicts the expression of the molecular markers, KRT13, GASP-1, and NXN2, associated with the aggressive prostate cancer cells established from cell culture of 3 human blood specimens, CTC-19, CTC-27 and CTC-9. These blood derived cells from prostate cancer patients expressed EMT (epithelial-to-mesenchymal transition), NE (neuroendocrine), sternness and prostate markers (A), and when stained by immunohistochemistry (IHC) show positive staining with KRT13, GASP-1 and NXN2 in cells, grown as 3-D spheroids, and as tumor xenografts.

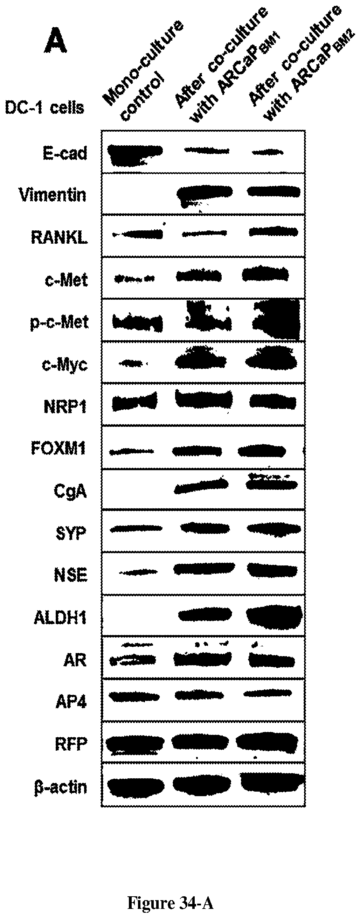

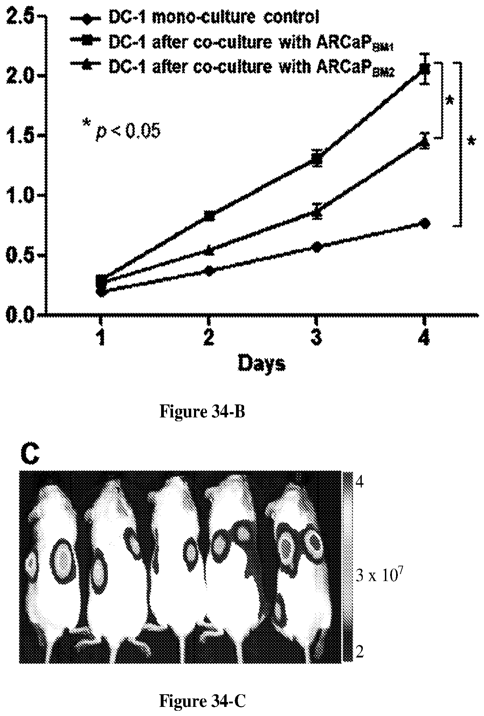

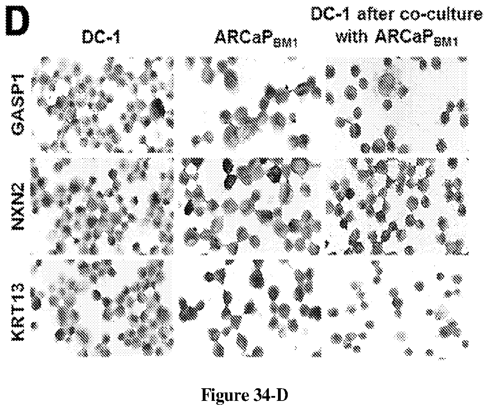

**Figure 34** depicts experimental confirmation of the interaction between indolent prostate cancer cells and aggressive leader cells as depicted in Figure 31. Co-culture of the aggressive ARCaPBM1 or ARCaPBM2 cells with indolent DC-1 cells confers the expression of a series of programmed genes, EMT, NE and sternness markers, to the indolent DC-1 cells (comparison to the monolayer culture with DC-1 cells, A). Behaviorally, DC-1 cells expressed the programmed genes, gained increased growth potential in cell culture (B), and as metastatic tumors in mice with tumor metastasis detected by bioluminescence of luciferase activity (C). Consistent with these behavioral changes, expression of three marker genes KRT13, GASP-1 and NXN2, associated with aggressive prostate cancer cells, is observed in programmed DC-1 cells (D).

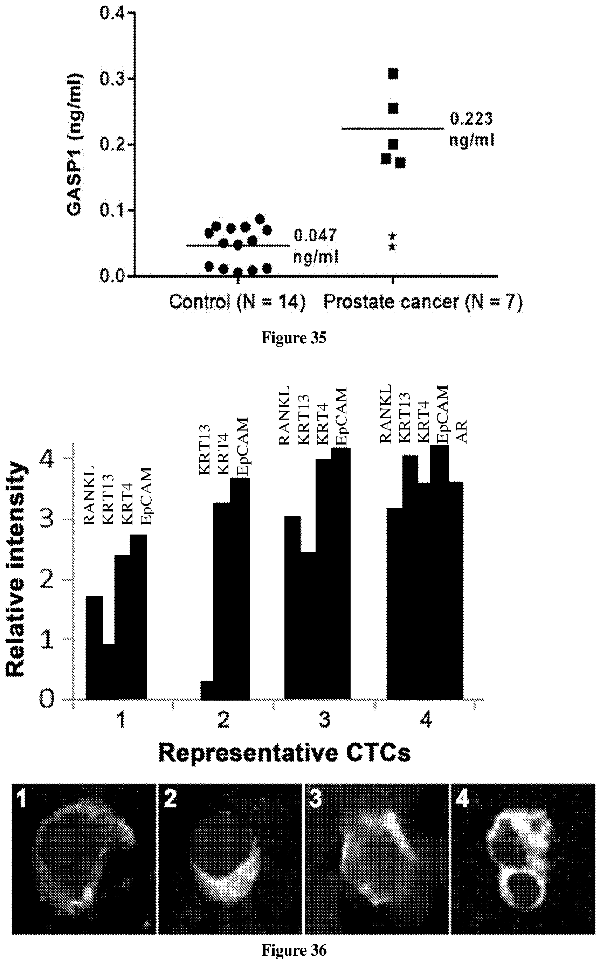

**Figure 35** depicts use of GASP-1 peptide as a companion diagnostic marker for human prostate cancer patients. Higher levels of GASP-1 peptide (average = 0.223 ng/ml) can be detected in prostate cancer patients than those of the controls (average = 0.047 ng/ml; the two stars designate patients with extreme small tumors who were determined to not have elevated GASP-1 peptide in their sera).
**Figure 36** depicts the heterogeneity of cultured circulating tumor cells (CTCs) from human prostate cancer patients based on their expression of RANKL, KRT13, KRT4, EpCAM and AR, which were determined by a multiplex quantum dot based semi-quantitative assay.

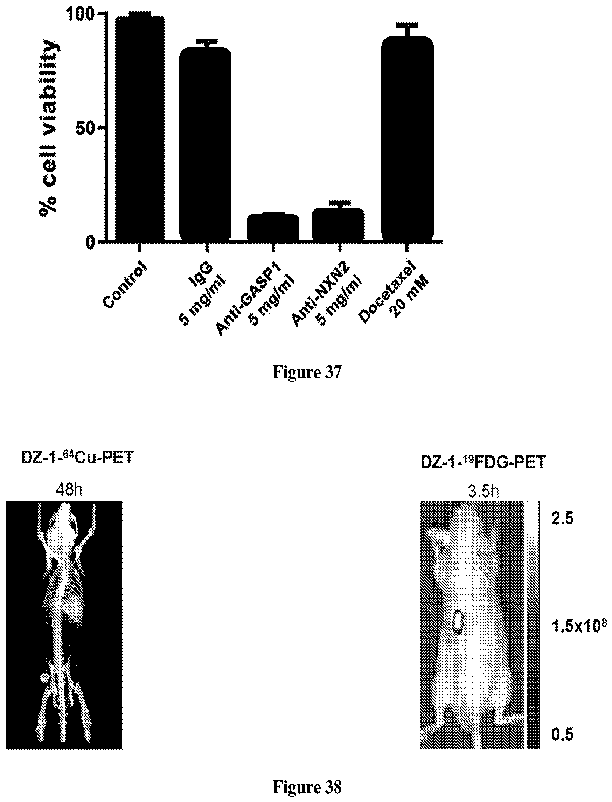

**Figure 37** depicts both the antibodies against GASP-1 and NXN2 exhibited cytotoxicity again the growth of docetaxel resistant prostate cancer cells in culture.
**Figure 38** depicts PET imaging of human lung tumors in mice using DZ1-18FDG, as demonstrated with non-radioactive DZ1-19FDG.

### DETAILED DESCRIPTION OF THE INVENTION

Unless defined otherwise, technical and scientific terms used herein have the same meaning as commonly understood by one of ordinary skill in the art to which this invention belongs. Singleton et al., Dictionary of Microbiology and Molecular Biology 3rd ed., Revised, J. Wiley & Sons (New York, NY 2006); March, Advanced Organic Chemistry Reactions, Mechanisms and Structure 7th ed., J. Wiley & Sons (New York, NY 2013) provide one skilled in the art with a general guide to many of the terms used in the present application.

One skilled in the art will recognize many methods and materials similar or equivalent to those described herein, which could be used in the practice of the present invention. Other features and advantages of the invention will become apparent from the following detailed description, taken in conjunction with the accompanying drawings, which illustrate, by way of example, various features of embodiments of the invention. Indeed, the present invention is in no way limited to the methods and materials described. For convenience, certain terms employed herein, in the specification, examples and appended claims are collected here.

Unless stated otherwise, or implicit from context, the following terms and phrases include the meanings provided below. Unless explicitly stated otherwise, or apparent from context, the terms and phrases below do not exclude the meaning that the term or phrase has acquired in the art to which it pertains. The definitions are provided to aid in describing particular embodiments, and are not intended to limit the claimed invention, because the scope of the invention is limited only by the claims. Unless otherwise defined, all technical and scientific terms used herein have the same meaning as commonly understood by one of ordinary skill in the art to which this invention belongs.

As used herein the term "comprising" or "comprises" is used in reference to compositions, methods, and respective component(s) thereof, that are useful to an embodiment, yet open to the inclusion of unspecified elements, whether useful or not. It will be understood by those within the art that, in general, terms used herein are generally intended as "open" terms (e.g., the term "including" should be interpreted as "including but not limited to," the term "having" should be interpreted as "having at least," the term "includes" should be interpreted as "includes but is not limited to," etc.).

Unless stated otherwise, the terms "a" and "an" and "the" and similar references used in the context of describing a particular embodiment of the application (especially in the context of claims) can be construed to cover both the singular and the plural. The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (for example, "such as") provided with respect to certain embodiments herein is intended merely to better illuminate the application and does not pose a limitation on the scope of the application otherwise claimed. The abbreviation, "e.g." is derived from the Latin exempli gratia, and is used herein to indicate a non-limiting example. Thus, the abbreviation "e.g." is synonymous with the term "for example." No language in the specification should be construed as indicating any non-claimed element essential to the practice of the application.

As used herein, the term "conjugate" refers to a compound formed by the joining of two or more compounds. Non-limiting examples of conjugates as described herein include dye-therapeutic moiety conjugates (dye-THM conjugates), NIR-dye-therapeutic moiety conjugates (NIR-dye-THM conjugates), dye-drug conjugates, NIR-dye-drug conjugates, dye-chemotherapy agent conjugates, NIR-dye-chemotherapy agent conjugates, dye-therapeutic agent conjugate. Non-limiting examples of conjugates as described herein include dye-simvastatin conjugate, dye-gemcitabine amide conjugate, dye-cisplatin amide conjugate, dye-cisplatin conjugate, dye-cisplatin conjugate with an ester bond, dye-artemisinin conjugate, NIR-dye- simvastatin conjugate, NIR-dye-gemcitabine amide conjugate, NIR-dye-cisplatin amide conjugate, NIR-dye-cisplatin conjugate, NIR-dye-cisplatin conjugate with an ester bond, NIR-dye-artemisinin conjugate, NIR-dye-therapeutic agent conjugate.

"THM" refers to a therapeutic moiety. Non-limiting examples of therapeutic moieties can be selected from any therapeutic agents including platinum based agents, chemotherapeutic agents, small molecules, peptides, proteins, polymers, siRNAs, microRNAs, nanoparticles, drugs, biologically active molecule, biologically active moiety. Non-limiting examples of therapeutic moieties can be selected from statin, (e.g., simvastatin, atorvastatin, fluvastatin, lovastatin, pitavastatin, pravastatin, rosuvastatin), cisplatin, oxoplatin, doxorubicin, artemisinin, and puromycin, and derivatives thereof. Non-limiting examples of therapeutic moieties can be selected from simvastatin, oxoplatin, doxorubicin, artemisinin, and puromycin, and derivatives thereof. Non-limiting examples of therapeutic moieties can be selected from simvastatin, oxoplatin, doxorubicin, dihydroartemisinin, and puromycin, and derivatives thereof.

Examples of dye-therapeutic moiety conjugates include without limitation,

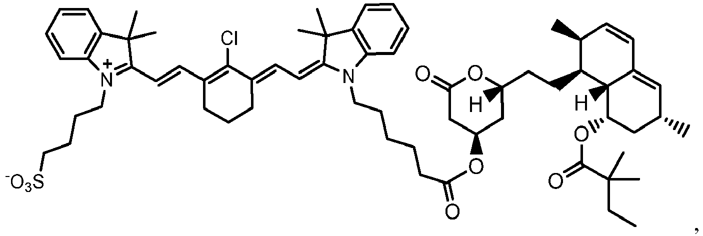

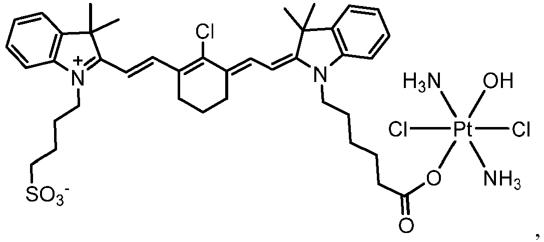

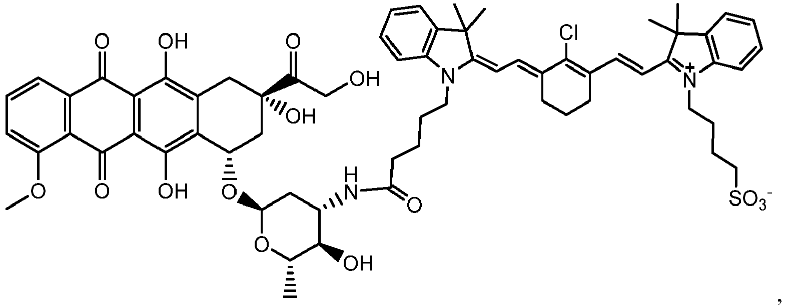

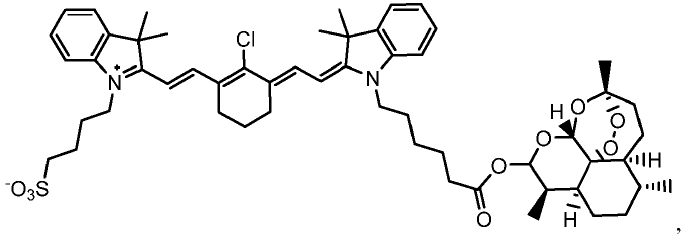

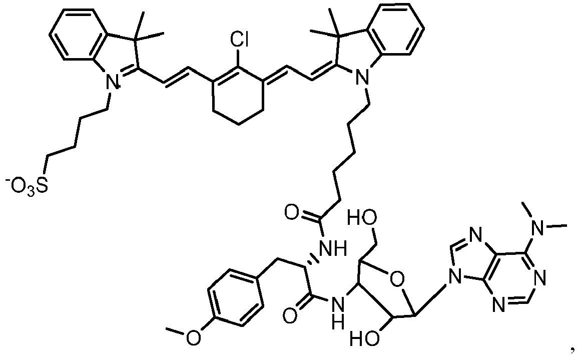

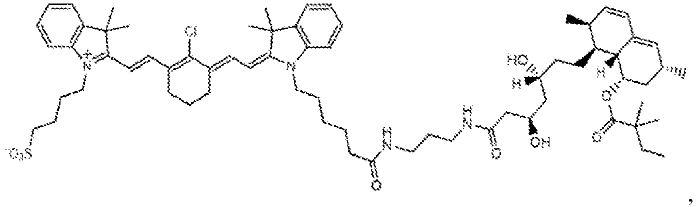

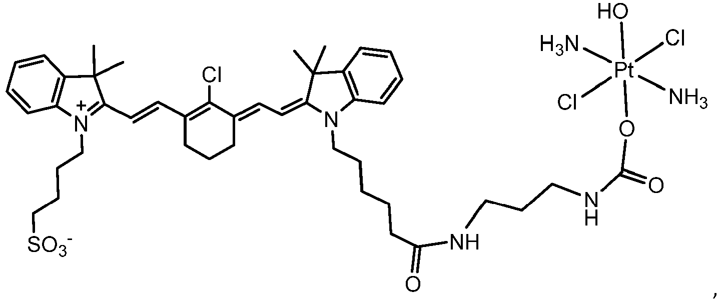

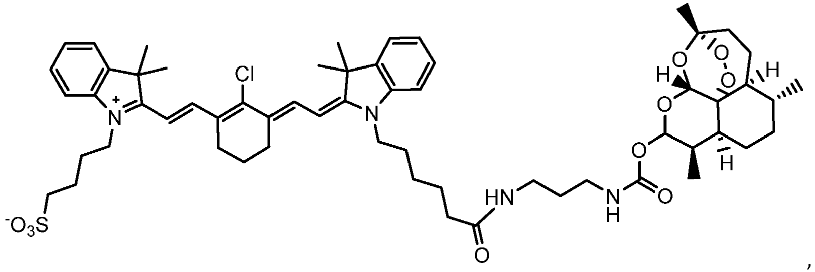

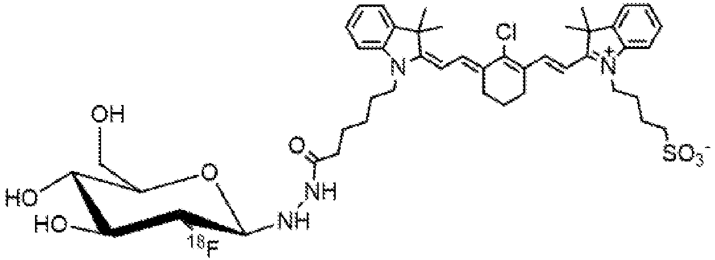

As used herein, the terms "alkyl," "alkenyl" and the prefix "alk-" are inclusive of both straight chain and branched chain groups and of cyclic groups, i.e. cycloalkyl and cycloalkenyl. Unless otherwise specified, these groups contain from 1 to 20 carbon atoms, with alkenyl groups containing from 2 to 20 carbon atoms. Preferred groups have a total of up to 10 carbon atoms. Cyclic groups can be monocyclic or polycyclic and can have from 3 to 10 ring carbon atoms. Exemplary cyclic groups include cyclopropyl, cyclopentyl, cyclohexyl, cyclopropylmethyl, adamantly, norbornane, and norbornene. This is also true of groups that include the prefix "alkyl-," such as alkylcarboxylic acid, alkyl alcohol, alkylcarboxylate, alkylaryl, and the like. Examples of suitable alkylcarboxylic acid groups are methylcarboxylic acid, ethylcarboxylic acid, and the like. Examples of suitable alkylacohols are methylalcohol, ethylalcohol, isopropylalcohol, 2-methylpropan-1-ol, and the like. Examples of suitable alkylcarboxylates are methylcarboxylate, ethylcarboxylate, and the like. Examples of suitable alkyl aryl groups are benzyl, phenylpropyl, and the like.

These may be straight chain or branched, saturated or unsaturated aliphatic hydrocarbon, which may be optionally inserted with N, O, or S. Representative saturated straight chain alkyls include methyl, ethyl, n-propyl, n-butyl, n-pentyl, n-hexyl, and the like; while saturated branched alkyls include isopropyl, sec-butyl, isobutyl, tert-butyl, isopentyl, and the like.

As used herein, the term "alkenyl" means an alkyl, as defined above, containing at least one double bond between adjacent carbon atoms. Alkenyls include both cis and trans isomers. Representative straight chain and branched alkenyls include ethylenyl, propylenyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, and the like.

As used herein, the term "alkynyl" means any alkyl or alkenyl, as defined above, which additionally contains at least one triple bond between adjacent carbons. Representative straight chain and branched alkynyls include acetylenyl, propynyl, 1-butynyl, 2-butynyl, 1-pentynyl, 2-pentynyl, 3-methyl-1 butynyl, and the like.

"Alkoxy" refers to an -OR group, wherein R is alkyl or substituted alkyl, preferably C1-C20 alkyl (e.g., methoxy, ethoxy, propyloxy, benzyloxy, etc.), most preferably C1 -C7.

"PEGyl" refers to a polyethylene chain with repeated moiety of (-CH₂-CH₂-O-)ₙ. n is ranging from 2 to 20. The remote end of the PEG is functionalized with amino, carboxylate, sulfonate, alkyne, sulfohydryl, hydroxyl, or any other functional group to enhance hydrophilicity or allow conjugation of compounds.

"Non-interfering substituents" are those groups that, when present in a molecule, are typically non-reactive with other functional groups contained within the molecule.

The term "substituted" as in, for example, "substituted alkyl" refers to a moiety (e.g., an alkyl group) substituted with one or more non-interfering substituents, such as, but not limited to: C3-C8 cycloalkyl, e.g., cyclopropyl, cyclobutyl, and the like; halo, e.g., fluoro, chloro, bromo, and iodo; cyano; alkoxy; phenyl; substituted phenyl, and the like.

"Aryl" as used herein includes carbocyclic aromatic rings or ring systems. As used herein, the term "aryl" refers to an aromatic 5-8 membered monocyclic, 8-12 membered bicyclic, or 11-14 membered tricyclic ring system. Examples of aryl groups include phenyl, naphthyl, biphenyl, fluorenyl and indenyl.

"Substituted aryl" is aryl having one or more non-interfering groups as a substituent. For substitutions on a phenyl ring, the substituents may be in any orientation (i.e., ortho, meta, or para).

"Heterocycle" or "heterocyclic" means one or more rings of 5-12 atoms, preferably 5-7 atoms, with or without unsaturation or aromatic character and having at least one ring atom which is not a carbon. Preferred heteroatoms include sulfur, oxygen, and nitrogen. Multiple rings may be fused, as in quinoline or benzofuran. Particularly preferred heterocycle groups are 5-10 membered rings with 1-3 heteroatoms selected from O, S, and N.

"Substituted heterocycle" is a heterocycle having one or more side chains formed from non-interfering substituents.

"Heteroaryl" is an aryl group containing from one to four N, O, or S atoms(s) or a combination thereof, which heteroaryl group is optionally substituted at carbon or nitrogen atom(s) with C1-6 alkyl, -CF₃, phenyl, benzyl, or thienyl, or a carbon atom in the heteroaryl group together with an oxygen atom form a carbonyl group, or which heteroaryl group is optionally fused with a phenyl ring.

Heteroaryl rings may also be fused with one or more cyclic hydrocarbon, heterocyclic, aryl, or heteroaryl rings. Heteroaryl includes, but is not limited to, 5-membered heteroaryls having one hetero atom (e.g., thiophenes, pyrroles, furans); 5-membered heteroaryls having two heteroatoms in 1,2 or 1,3 positions (e.g., oxazoles, pyrazoles, imidazoles, thiazoles, purines); 5-membered heteroaryls having three heteroatoms (e.g., triazoles, thiadiazoles); 5-membered heteroaryls having 3 heteroatoms; 6-membered heteroaryls with one heteroatom (e.g., pyridine, quinoline, isoquinoline, phenanthrine, 5,6- cycloheptenopyridine); 6-membered heteroaryls with two heteroatoms (e.g., pyridazines, cinnolines, phthalazines, pyrazines, pyrimidines, quinazolines); 6-membered heretoaryls with three heteroatoms (e.g., 1,3,5-triazine); and 6-membered heteroaryls with four heteroatoms. Particularly preferred heteroaryl groups are 5-10-membered rings with 1-3 heteroatoms selected from O, S, and N.

"Substituted heteroaryl" refers a heteroaryl having one or more non-interfering groups as substituents.

Each of the terms "drug," "biologically active molecule," "biologically active moiety," "active agent" and "biologically active agent", when used herein, means any substance which can affect any physical or biochemical properties of a biological organism, including but not limited to viruses, bacteria, plants, animals, and humans. In particular, as used herein, biologically active molecules include any substance intended for diagnosis, cure, mitigation, treatment, or prevention of disease in humans or other animals, or to otherwise enhance physical or mental well-being of humans or animals. Examples of biologically active molecules include, but are not limited to, peptides, proteins, enzymes, small molecule drugs, dyes, lipids, nucleosides, oligonucleotides, polynucleotides, nucleic acids, cells, viruses, liposomes, microparticles and micelles. Classes of biologically active agents that are suitable for use with the invention include, but are not limited to, antibiotics, fungicides, anti-viral agents, antiinflammatory agents, anti-tumor agents, cardiovascular agents, anti-anxiety agents, hormones, growth factors, steroidal agents, and the like.

"Pharmaceutically acceptable excipient" or "pharmaceutically acceptable carrier" refers to an excipient that can be included in the compositions of the invention and that causes no significant adverse toxicological effects to the patient.

"Pharmacologically effective amount," "physiologically effective amount," and "therapeutically effective amount" are used interchangeably herein to mean the amount of an agent and/or conjugate of the invention present in a pharmaceutical preparation that is needed to provide a desired level of active agent and/or conjugate in the bloodstream or in the target tissue. The precise amount will depend upon numerous factors, e.g., the particular active agent, the components and physical characteristics of the pharmaceutical preparation, intended patient population, patient considerations, and the like, and can readily be determined by one skilled in the art, based upon the information provided herein and available in the relevant literature.

The term "prodrug" refers to a compound that formulated as a precursor compound that, following administration, activates or releases the active component of the compound in vivo via a chemical or physiological process (e.g., upon being brought to physiological pH or through enzyme activity). A discussion of the synthesis and use of prodrugs is provided by Higuchi and Stella, Prodrugs as Novel Delivery Systems, vol. 14 of the ACS Symposium Series, and Bioreversible Carriers in Drug Design, ed. Edward B. Roche, American Pharmaceutical Association and Pergamon Press, 1987. Accordingly, the term "prodrug" refers to compounds that can be converted via some chemical or physiological process (e.g., enzymatic processes and metabolic hydrolysis) to an inactive form that can be activated in vivo by some co-compound or a specific environmental condition, e.g., pH etc. A prodrug may be inactive when administered to a subject, i.e. an ester, but is converted in vivo to an active compound, for example, by hydrolysis to the free carboxylic acid or free hydroxyl. The prodrug compound often offers advantages of solubility, tissue compatibility or delayed release in an organism. The term "prodrug" is also meant to include any covalently bonded carriers, which release the active compound in vivo when such prodrug is administered to a subject.

An "active metabolite" is a physiologically active compound which results from the metabolism of a compound of the invention, or a prodrug thereof, when such compound or prodrug is administered to a mammal.

"Polypeptide" or "poly(amino acid)" refers to any molecule comprising a series of amino acid residues, typically at least about 5-20 residues, linked through amide linkages (also referred to as peptide linkages) along the alpha carbon backbone. While in some cases the terms may be used synonymously herein, a polypeptide is a peptide typically having a molecular weight up to about 10,000 Da, while peptides having a molecular weight above that are commonly referred to as proteins. Modifications of the peptide side chains may be present, along with glycosylations, hydroxylations, and the like. Additionally, other non-peptidic molecules, including lipids and small drug molecules, may be attached to the polypeptide. The polypeptide may comprise any combination or sequence of amino acid residues. The polymers of the invention are suitable for covalent attachment to both polypeptides and proteins.

"Amino acid" refers to organic acids containing both a basic amine group and an acidic carboxyl group. The term encompasses essential and non-essential amino acids and both naturally occurring and synthetic or modified amino acids. The most common amino acids are listed herein by either their name or by the three letter or single letter abbreviations: Glycine (Gly, G), Alanine (Ala, A), Valine (Val, V), Leucine (Leu, L), Isoleucine (Ile, I), Methionine (Met, M), Proline (Pro, P), Phenylalanine (Phe, F), Tryptophan (Trp, W), Serine (Ser, S), Threonine (Thr, T), Asparagine (Asn, N), Glutamine (Gln, Q), Tyrosine, (Tyr, Y), Cysteine (Cys, C), Lysine (Lys, K), Arginine (Arg, R), Histidine (His, H), Aspartic Acid (Asp, D), and Glutamic acid (Glu, E).

By "residue" is meant the portion of a molecule remaining after reaction with one or more molecules. For example, an amino acid residue in a polypeptide chain is the portion of an amino acid remaining after forming peptide linkages with adjacent amino acid residues.

"Electron withdrawing group" or EWG refers to functional groups that remove electron density from the ring by making it less nucleophilic. This class can be recognized by the atom adjacent to the 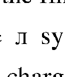 system having several bonds to more electronegative atoms or the presence of a formal charge. Examples of these groups include halogens, aldehydes, ketones, esters, carboxylic acids, acid chlorides, nitriles, nitrosos, and sulfonic acids.

"Electron donating group" or EDG refers to functional groups that add electron density to the ring by making it more nucleophilic. This class can be recognized by lone pairs on the atom adjacent to the 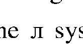 system. Examples of these groups include alkyl, alkenyl, alkynyl, amides, ethers, alkoxides, alcohols, and amines.

The term "patient," refers to a living organism suffering from or prone to a condition that can be prevented or treated by administration of an agent or conjugate of the invention (including various embodiments thereof), and includes both humans and animals.

"Optional" or "optionally" means that the subsequently described circumstance may or may not occur, so that the description includes instances where the circumstance occurs and instances where it does not.

In various embodiments, compounds of the invention are dye-therapeutic moiety conjugates.

### Methods of Using the Dye-Therapeutic Moiety Conjugates of the Invention

The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

The compounds of the invention are believed to act as agents with preferential entry and retention in cancer cells in vivo upon ambient administration. It is believed that the compounds are, therefore, useful to identify and treat or prevent or reduce the likelihood of having any condition responsive to the preferential entry and retention properties of the compounds of the invention.

Subjects which can be imaged or treated include animal subjects, typically vertebrates, including both mammalian (e.g., human, cat, dog, cow, horse, sheep, pig, monkey, ape, etc.) and avian subjects (e.g., chicken, turkey, duck, goose, quail, pheasant, etc.).

For example, the compounds of the present invention can be used in the treatment of cancerous tissue and the tumors associated therewith, including without limitation breast, lung, pancreatic, renal, ovarian, brain, colon, prostate and skin cancer.

Administering an effective amount of a compound of the invention to a subject can result in detection of cancerous tissue. Additionally, the same or other compounds of this invention can be cytotoxic in cancerous tissue. Thus, provided are methods for detecting and treating tumor-bearing subjects in which the compounds of the invention are administered to the subject in need of such treatment in a manner effective to identify and/or treat such tumors.

By "treatment or prevention" the term is intended to refer to the alleviation of the signs, symptoms, or causes of a disease, or any other desired alteration of a biological system. Accordingly, the method "prevents" (i.e., delays or inhibits) and/or "reduces" (i.e., decrease, slows, or ameliorates) the detrimental effects of the cancer, or neoplastic disease or disorder, in the mammal receiving the therapy.

As used herein, a "neoplastic disease or disorder" is characterized by one or more of the following properties: cell growth that is not regulated by the normal biochemical and physical influences in the environment; anaplasia (i.e., lack of normal coordinated cell differentiation); and in some instances, metastasis. Further, as used herein, the term "cancer" is understood to mean a disease characterized by abnormal growth of cells that is not regulated by the normal biochemical and physical influences in the environment. Accordingly, as used herein, the terms cancer and neoplasia are intended to be interchangeable.

Neoplastic diseases capable of treatment according to the invention include, for example, anal carcinoma, bladder carcinoma, breast carcinoma, cervix carcinoma, chronic lymphocytic leukemia, chronic myelogenous leukemia, endometrial carcinoma, hairy cell leukemia, head and neck carcinoma, lung (small cell) carcinoma, multiple myeloma, non-Hodgkin's lymphoma, follicular lymphoma, ovarian carcinoma, brain tumors, colorectal carcinoma, hepatocellular carcinoma, Kaposi's sarcoma, lung (non- small cell carcinoma), melanoma, pancreatic carcinoma, prostate carcinoma, renal cell carcinoma, ductal carcinoma, gastric carcinoma, squamous cell carcinoma, basal cell carcinoma, and soft tissue sarcoma. Additional neoplastic disorders can be found in, for example, Isselbacher et al. (1994) Harrison S Principles of Internal Medicine 1814- 1877.

Delivery of a therapeutically effective activity of a compound of the invention can be obtained via administration of a pharmaceutical composition comprising a therapeutically effective activity of a compound of the invention. By "therapeutically effective activity" or "dose" is meant a concentration of a labeled construct of the invention that is sufficient to elicit the desired therapeutic effect according to the various methods of treatment described herein. Accordingly, in one embodiment, a therapeutically effective activity is an activity effective to treat cancer, such as inhibiting or slowing growth of cancerous tissue. The therapeutically effective dosage of any specific compound will vary somewhat from compound to compound, patient to patient, and will depend upon the condition of the patient and the route of delivery. The effective activity of any particular compound would be expected to vary according to the weight, sex, age, and medical history of the subject. Other factors which influence the effective dose can include, but are not limited to, the severity of the patient's condition, the disease or disorder being treated, the stability of the compound according to the invention, and, if appropriate, any additional antineoplastic therapeutic agent being administered with the compound of the invention. Methods to determine efficacy and dosage are known to those skilled in the art. See, for example, Isselbacher et al. (1996) Harrison S Principles of Internal Medicine 13 ed., 1814-1882.

Effective amounts, toxicity, and therapeutic efficacy can be determined by standard pharmaceutical procedures in cell cultures or experimental animals, e.g., for determining the LD50 (the dose lethal to 50% of the population) and the ED50 (the dose therapeutically effective in 50% of the population). The dosage can vary depending upon the dosage form employed and the route of administration utilized. The dose ratio between toxic and therapeutic effects is the therapeutic index and can be expressed as the ratio LD50/ED50. In some embodiments, compositions exhibit large therapeutic indices. A therapeutically effective dose can be estimated initially from cell culture assays. Also, a dose can be formulated in animal models to achieve a circulating plasma concentration range that includes the IC50 (*i.e*., the concentration of the compound of the invention, which achieves a half-maximal inhibition of symptoms) as determined in cell culture, or in an appropriate animal model. Levels in plasma can be measured, for example, by high performance liquid chromatography. The effects of any particular dosage can be monitored by a suitable bioassay. The dosage can be determined by a physician and adjusted, as necessary, to suit observed effects of the treatment.

In certain embodiments, an effective dose of a composition comprising a compound of the invention can be administered to a patient to identify, image or localize cancerous cells and tumors. In some embodiments, a compound of the invention can be administered to a patient once. In certain embodiments, an effective dose of a composition comprising a compound of the invention can be administered to a patient repeatedly. Patients can be administered a therapeutic amount of a composition comprising a compound of the invention. A composition comprising a compound of the invention can be administered over a period of time, such as over a 5 minute, 10 minute, 15 minute, 20 minute, or 25 minute period. If warranted, the administration can be repeated, for example, on a regular basis, such as hourly for 3 hours, 6 hours, 12 hours or longer or such as biweekly (i.e., every two weeks) for one month, two months, three months, four months or longer. In some instances, after an initial treatment regimen, the treatments can be administered on a less frequent basis. For example, after administration biweekly for three months, administration can be repeated once per month, for six months or a year or longer.

The dosage of a composition as described herein can be determined by a physician and adjusted, as necessary, to suit observed effects of the treatment. With respect to duration and frequency of treatment, it is typical for skilled clinicians to monitor subjects in order to determine when the treatment is providing therapeutic benefit, and to determine whether to increase or decrease dosage, increase or decrease administration frequency, discontinue treatment, resume treatment, or make other alterations to the treatment regimen. The dosing schedule can vary from once a week to daily depending on a number of clinical factors, such as the subject's sensitivity to the compound of the invention.

In certain embodiments, an effective dose of a composition comprising a compound of the invention can be administered to treat, inhibit, or prevent a cancer in a patient. In certain embodiments, an effective dose of a composition comprising a compound of the invention as described herein can be administered to a patient once. In certain embodiments, an effective dose of a composition comprising a compound of the invention can be administered to a patient repeatedly. In certain embodiments, an effective dose of a composition comprising a compound of the invention can be administered to a patient via skin patches, instillation into urinary bladder through urethral, or vaginal channel through ring implantation. Patients can be administered a therapeutic amount of a composition comprising a compound of the invention. A composition comprising a compound of the invention can be administered over a period of time, such as over a 5 minute, 10 minute, 15 minute, 20 minute, or 25 minute period. If warranted, the administration can be repeated, for example, on a regular basis, such as hourly for 3 hours, 6 hours, 12 hours or longer or such as biweekly (i.e., every two weeks) for one month, two months, three months, four months or longer. In some instances, after an initial treatment regimen, the treatments can be administered on a less frequent basis. For example, after administration biweekly for three months, administration can be repeated once per month, for six months or a year or longer. Administration of a composition comprising compound of the invention can reduce levels of a biomarker or a symptom of cancer.

The dosage of a composition as described herein can be determined by a physician and adjusted, as necessary, to suit observed effects of the treatment. With respect to duration and frequency of treatment, it is typical for skilled clinicians to monitor subjects in order to determine when the treatment is providing therapeutic benefit, and to determine whether to increase or decrease dosage, increase or decrease administration frequency, discontinue treatment, resume treatment, or make other alterations to the treatment regimen. The dosing schedule can vary from once a week to daily depending on a number of clinical factors, such as the subject's sensitivity to the compound of the invention.

Embodiments of the present invention related to the use of carbocyanine dyes, particularly, dye-therapeutic moiety conjugates, include but not limited to study the in situ pharmacokinetics and pharmacodynamics of pharmaceuticals in live animals and humans. This application is important and significant because until now, pharmacokinetics and pharmacodynamics are measured using bodily fluids available for detection. The ability of the dye-therapeutic moiety conjugates to be specifically taken up by tumor tissues could lead to methods of determining the in situ pharmacokinetics and pharmacodynamics of clinically useful imaging agents and therapeutic drugs in the body and at tumor sites. Dye-therapeutic moiety conjugates can be used as a chemical tag for tumor cells. The tagged tumor cells can be isolated by FACS sorting. The isolated tumor cells from biological fluids can be further characterized. Dye-therapeutic moiety conjugates can also be used to tag stem cells, putative tumor stem cells or any biologicals that can penetrate organ, tissue or cellular compartment for improved real-time imaging.

Dye-therapeutic moiety conjugates can be chemically conjugated to drugs, organic and inorganic molecules with ligands known to bind to normal cells for normal organ, tissue repair and regeneration. Because of its unique property, it can be visualized at the site of drug action and the biologic response of the tissues and organs to these reagents can be assessed and correlated.

Dye-therapeutic moiety conjugates can be used as an agent to image and treat patients on a personalized basis. It is known that drug accumulation and metabolism in individual patients are different, but unfortunately it is difficult to monitor the pharmacokinetic and pharmacodynamic properties of drugs with desirable precision. Dye-therapeutic moiety conjugates could solve this problem because this compound can be imaged at tumor sites, thus providing vital information on the pharmacokinetic and pharmacodynamic properties of drugs.

GASP-1 and NXN2 may be used as companion biomarkers for the cancer types described herein.

A method as described herein may further comprise the step of determining the concentration of one or more companion biomarker, in particular, of a GASP-1 peptide or a NXN2 peptide, in one or more of fluid, blood serum, and tissue of the patient. Preferably such determination is performed before administration of the dye-therapeutic moiety conjugate to the patient. Thus, advantageously, the cancer types and patients that benefit from use of the dye-therapeutic conjugates may be identified at a very early stage, before progression of the disease. The companion biomarkers may be useful for any cancer, including patients with prostate, pancreatic, lung or kidney cancer, and may be particularly useful patients presenting with prostate cancer, and in particular docetaxel resistant prostate cancer. In any of these patients, GASP-1 and/or NXN2 may be elevated, and such elevation may identify the presence of cancer, and start of administration of the dye-therapeutic conjugate and/or cancer drug. For example, the based on determination of the GASP-1 or NXN2 concentration, for example by ELISA as described in **Example 16**, a dye-therapeutic moiety conjugate as described herein may be administered to the patient, e.g. for therapy or imaging. The elevation may be determined in one or more ways, including a comparison to a control established from non-cancer patients, and a comparison of serum specimens obtained from the same patient longitudinally. Longitudinally means obtaining a serum sample from a patient at different time, for example, before and after surgery, before and after treatment, or before and after radiographical determination of tumor progression or regression. Longitudinal determination may thus confirm reliability and/or serve as a standard for serum GASP-1, to determine e.g. cancer presence, cancer progression or regression. The elevation may be an increase of, for example,about 2 to about 100 fold increased, e.g. about 5 fold to about 75fold or typically about 10 to about 30 fold increase.

Dye-therapeutic moiety conjugates can be used to tag circulating stem cells for the assessment of stem cell grafting of bone marrow or other vital organs. Normal cells can have a low rate of uptake and this can be improved by the use of internalized cell surface ligand in conjugation with dye-therapeutic moiety conjugates, which could potentially increase the amount of uptake into stem cell populations.

This same concept can be applied by the conjugation of dye-therapeutic moiety conjugates to other tissue and organ-specific cell surface molecules for potential imaging and cytotoxicity purposes. For example, dye-therapeutic moiety conjugates can be guided into human BPH tissues using a prostate cell surface ligand plus cytotoxic drug conjugates for tumor ablation.

Likewise, a number of benign tumors which can be difficult for surgical approaches can also be candidates for selective tumor ablation using dye-therapeutic moiety conjugates as the imaging and cytotoxic agent.

Various embodiments provide for pharmaceutical composition comprising an dye-therapeutic moiety conjugates of the present invention and a pharmaceutically acceptable carrier.

In one aspect of the present invention, pharmaceutical compositions are provided, which comprise one or more (e.g. 1, 2, 3, 4, 5, 6, 7, 8, 9, 10, or more) of the compounds described herein (or a prodrug, pharmaceutically acceptable salt, or other pharmaceutically acceptable form thereof), and optionally a pharmaceutically acceptable excipient. In certain embodiments, these compositions optionally further comprise one or more additional therapeutic agents. Alternatively, a compound of the invention can be administered to a patient in need thereof in combination with the administration of one or more other therapeutic agents. For example, in the regulation of skin pigmentation, an additional therapeutic agent for conjoint administration or inclusion in a pharmaceutical composition with a compound of this invention can be an approved skin pigmentation agent.

Amount of the compound or compounds in the pharmaceutical composition can be based on weight, moles, or volume. In some embodiments, the pharmaceutical composition comprises at least 0.0001% compounds of the invention. In some embodiments, the pharmaceutical composition comprises at least 0.1% compounds of the invention. In some embodiments, the pharmaceutical composition comprises at least 0.5% compounds of the invention. In some embodiments, the pharmaceutical composition comprises at least 1% compounds of the invention. In some embodiments, the pharmaceutical composition comprises at least 2% compounds of the invention. In some embodiments, the pharmaceutical composition comprises at least 3% compounds of the invention. In some embodiments, the pharmaceutical composition comprises at least 4% compounds of the invention. In some embodiments, the pharmaceutical composition comprises at least 5% compounds of the invention. In some embodiments, the pharmaceutical composition comprises at least 10% compounds of the invention. In some embodiments, the pharmaceutical composition comprises 0.01%-99% of the compounds of the invention. In some embodiments, the pharmaceutical composition comprises 0.05%-90% of the compounds of the invention. In some embodiments, the pharmaceutical composition comprises 0.1%-85% of the compounds of the invention. In some embodiments, the pharmaceutical composition comprises 0.5%-80% of the compounds of the invention. In some embodiments, the pharmaceutical composition comprises 1%-75% of the compounds of the invention. In some embodiments, the pharmaceutical composition comprises 2%-70% of the compounds of the invention. In some embodiments, the pharmaceutical composition comprises 3%-65% of the compounds of the invention. In some embodiments, the pharmaceutical composition comprises 4%-60% of the compounds of the invention. In some embodiments, the pharmaceutical composition comprises 5%-50% of the compounds of the invention.

Provided is a method of identifying, imaging or localizing cancerous cells and tumors in a patient in need thereof. The method can comprise providing the dye-therapeutic moiety conjugates of the present invention; administering the dye-therapeutic moiety conjugate to the patient; and performing optical imaging.

Provided is a method of identifying, imaging or localizing cancerous cells and tumors in a patient in need thereof. The method can comprise providing the dye-therapeutic moiety conjugates of the present invention; administering the dye-therapeutic moiety conjugates to the patient; and imaging the tumor location within the NIR spectral region. In various embodiments, imaging the tumor is performed about 6 to 48 hours post injection.

Provided is a method of treating or preventing cancer in a patient in need thereof. The method can comprise providing the dye-therapeutic moiety conjugates of the present invention; and administering the dye-therapeutic moiety conjugates to the patient.

Provide is a method of treating or preventing cancer in a patient in need thereof. The method can comprise providing the dye-therapeutic moiety conjugates of the present invention; and administering the dye-therapeutic moiety conjugates to the patient.

The compositions described herein can be administered to a subject having or diagnosed as having cancer. In some embodiments, the methods described herein comprise administering an amount of compositions described herein to a subject in order to treat or prevent cancer. For example, as compared with an equivalent untreated control, a reduction of a symptom by at least 5%, 10%, 20%, 40%, 50%, 60%, 80%, 90%, 95%, 99% or more as measured by any standard technique. A variety of means for administering the compositions described herein to subjects are known to those of skill in the art. Such methods can include, but are not limited to oral or parenteral routes, including intravenous, intramuscular, subcutaneous, transdermal, airway (aerosol), pulmonary, or intratumoral. Administration can be local or systemic.

The term "effective amount" as used herein refers to the amount compound of the instant invention needed to alleviate the cancer, and relates to a sufficient amount of pharmacological composition to provide the desired effect. The term "therapeutically effective amount" therefore refers to an amount of a compound of the invention that is sufficient to cause a particular anti-cancer effect when administered to a typical subject. An effective amount as used herein, in various contexts, would also include an amount sufficient to delay the development of a symptom of the disease, alter the course of a symptom disease (for example but not limited to, slow the progression of a symptom of the disease), or reverse a symptom of the disease. Thus, it is not generally practicable to specify an exact "effective amount". However, for any given case, an appropriate "effective amount" can be determined by one of ordinary skill in the art using only routine experimentation.

Effective amounts, toxicity, and therapeutic efficacy can be determined by standard pharmaceutical procedures in cell cultures or experimental animals, e.g., for determining the LD50 (the dose lethal to 50% of the population) and the ED50 (the dose therapeutically effective in 50% of the population). The dosage can vary depending upon the dosage form employed and the route of administration utilized. The dose ratio between toxic and therapeutic effects is the therapeutic index and can be expressed as the ratio LD50/ED50. In some embodiments, compositions exhibit large therapeutic indices. A therapeutically effective dose can be estimated initially from cell culture assays. Also, a dose can be formulated in animal models to achieve a circulating plasma concentration range that includes the IC50 (i.e., the concentration of the compound of the invention, which achieves a half-maximal inhibition of symptoms) as determined in cell culture, or in an appropriate animal model. Levels in plasma can be measured, for example, by high performance liquid chromatography. The effects of any particular dosage can be monitored by a suitable bioassay, e.g., assay for inhibition of cancer cell proliferation, among others. The dosage can be determined by a physician and adjusted, as necessary, to suit observed effects of the treatment.

The dosage of a composition as described herein can be determined by a physician and adjusted, as necessary, to suit observed effects of the treatment. With respect to duration and frequency of treatment, it is typical for skilled clinicians to monitor subjects in order to determine when the treatment is providing therapeutic benefit, and to determine whether to increase or decrease dosage, increase or decrease administration frequency, discontinue treatment, resume treatment, or make other alterations to the treatment regimen. The dosing schedule can vary from once a week to daily depending on a number of clinical factors, such as the subject's sensitivity to the compound of the invention.

Various embodiments provide for a method for the detection of a molecule in tumor or normal cells, or tumor or normal tissue. The method can comprise providing the dye-therapeutic moiety conjugates of the present invention;; and imaging the tumor or normal cells, or tumor or normal tissue.

Various embodiments provide for a method of conducting in situ pharmacokinetic and pharmacodynamic analyses of the dye-therapeutic moiety conjugates of the present invention and its drug payload in a tumor or normal cell or tissue. The method can comprise providing the dye-therapeutic moiety conjugates of the present invention and its drug payload; contacting the dye-therapeutic moiety conjugates of the present invention and its drug payload with the tumor or normal cell or tissue; and imaging the tumor or normal cell or tissue.

Provided is a method of conducting surgical removal of a tumor in a patient in need thereof. The method can comprise providing the dye-therapeutic moiety conjugates of the present invention; administering the dye-therapeutic moiety conjugates to the patient; imaging the patient to detect cancer cells in situ at surgical margin at the time of surgery; and performing the surgical removal of the tumor.

Various embodiments provide for a method to tag a cell, microorganism and small molecule. The method can comprise providing the dye-therapeutic moiety conjugates of the present invention; contacting the dye-therapeutic moiety conjugates with the cell, microorganism, or small molecule; and imaging the dye-therapeutic moiety conjugates with the cell, microorganism, or small molecule to follow its movements in live subjects.

Provided is a method of studying cancer metastases in conventional or transgenic animal. The method can comprise providing the dye-therapeutic moiety conjugates of the present invention; injecting the dye-therapeutic moiety conjugates into a conventional or transgenic animal bearing either a mouse or human tumor; and monitoring the tumor by using optical imaging technology.

Various embodiments of the present invention also provide for methods of synthesizing the dye-therapeutic moiety conjugates described in more detail in the schemes and the examples below.

### EXAMPLES

The following examples are not intended to limit the scope of the claims to the invention, but are rather intended to be exemplary of certain embodiments. Any variations in the exemplified methods which occur to the skilled artisan are intended to fall within the scope of the present invention.

.

### Materials and Methods

Simvastatin was purchased from Ark Pharm, Inc. (Arlington Heights, IL). DZ-1 (1) for the current studies was synthesized as reported earlier. Cisplatin and doxorubicin were purchased from MedKoo Biosciences, Inc. (Chapel Hill, NC). Puromycin dihydrochloride was purchased from Alfa Aesar (Tewksbury, MA). Dihydroartemisinin was purchased from TCI America (Portland, OR). All other chemicals and reagents were purchased from standard sources such as Sigma-Aldrich and/or VWR and were of highest quality available. Deionized water (18.2 Ω) used for making solutions was obtained from Milli-Q Direct Ultrapure Water System from Millipore (Billerica, MA, USA). All intermediates were characterized by 1H NMR and mass analysis and the purity of compounds were analyzed by HPLC. 1H NMR data were collected on Bruker 400MHz spectrometers using standard parameters; chemical shifts are reported in ppm (δ) in reference to residual non-deuterated solvent. ESI mass spectroscopy analysis was performed on new compounds at Mass Spectrometry and Biomarker Discovery Core facility using a Thermo Fisher LTQ Orbitrap Elite system at the Cedars-Sinai Medical Center.

### Reference Example 1.

**Synthesis of DZ-1-simvastatin conjugate 3:** DZ-1 **1** (407 mg, 0.58 mmol), simvastatin **2** (387 mg, 0.92 mmol), 1-ethyl-3-(3-dimethyllaminopropyl) carbodiie hydrochloride (172 mg, 0.89) and DMAP (65 mg, 0.53mmol) were mixed and dissolved in anhydrous methylene chloride (15 mL). The resulted mixture was stirred for 18 h. The reaction mixture was concentrated on a rotary evaporator. The residue was dissolved in 5mL of methanol and purified by C18-RP silica chromatography elution with methanol-water to afford desired product **3** as a dark green solid (269 mg, yield 42%). 1H NMR (DMSO-d6, 400 MHz) δ 8.23 (m, 2H), 7.61-7.56(m, 2H), 7.47(d, 1H), 7.42-7.36(m, 3H), 7.29-7.19 (m, 2H), 6.4-0 (d, 1H), 6.24 (d, 1H), 5.89(d, 1H),5.73(m, 1H), 5.71(s, 1H), 5.43(m, 1H), 5.12-5.07(m, 2H), 4.32(m, 1H), 4.21 (m, 2H), 4.14 (m, 2H), 2.82 (m, 1H), 2.69 (m, 4H), 2.27 (m, 4H), 1.83 (m, 6H), 1.70 (m, 3H), 1.63(s, 6H), 1.62(s, 6H), 1.53 (m, 6H), 1.38 (m, 6H), 1.23 (m, 4H), 1.14 (m, 4H), 1.01(s, 1H), 0.97(s, 6H), 0.77(q, 2H), 0.67(t, 3H). Mass spectrum (ESI) m/z 1105.58 [M+H]+.

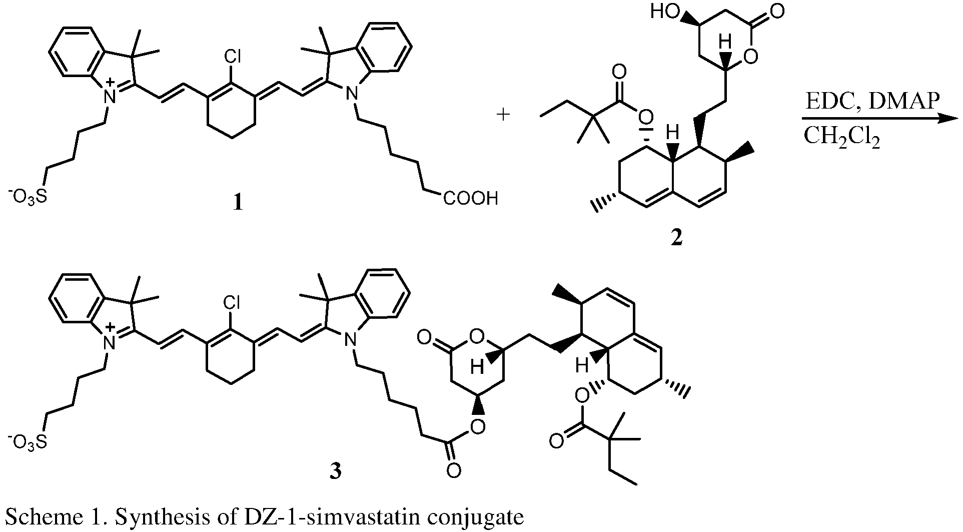

### Reference Example 2.

**Synthesis of DZ-1-cisplatin conjugate 6:** The detailed chemical synthesis of Dye-cisplatin conjugate is described in scheme 2. Oxoplatin **5** was synthesized according to the literature method (Journal of Inorganic Biochemistry 107 (2012) 6-14). To a suspension of compound **5** (350 mg, 1.05 mmol) in DMSO (20 mL) was added DZ-1 **1** (500 mg, 0.71 mmol) followed by 1-ethyl-3-(3-dimethyllaminopropyl) carbodiie hydrochloride (136 mg, 0.0.71) and DMAP (80 mg, 0.65 mmol) and the mixture was stirred for 20h at room temperature to afford green solution. The solution was filtered and the DMSO was removed by lyophilization. The product was purified with C18-RP silica chromatography, elution with methanol-water to afford DZ-1-cisplatin **6** as a dark green solid (275 mg, yield 38%). 1H NMR (DMSO-d6, 400 MHz) δ 8.22 (m, 2H), 7.61-7.56(m, 2H), 7.48(d, 1H), 7.39(m, 3H), 7.26 (m, 2H), 6.37 (d, 1H), 6.24 (d, 1H), 4.15 (m, 4H), 2.68 (m, 4H), 2.15 (m, 2H), 1.80 (m, 4H), 1.70 (m, 6H), 1.63(s, 6H), 1.62(s, 6H), 1.51 (m, 2H), 1.40 (m, 2H). Mass spectrum (ESI) m/z 1020.69 (M + H)+.

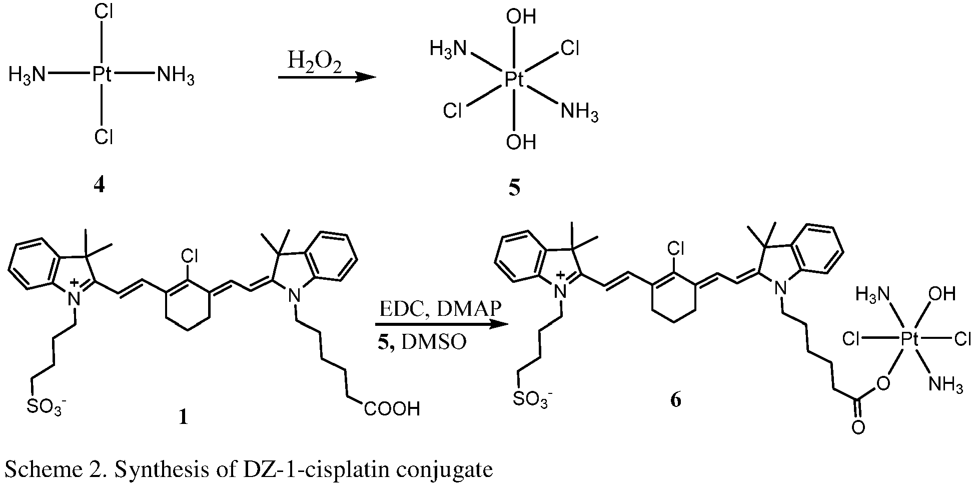

### Reference Example 3.

**Synthesis of DZ-1-doxorubicin conjugate 7:** The mixture of DZ-1 **1** (103 mg, 0.15 mmol) 1-ethyl-3-(3-dimethyllaminopropyl) carbodiie hydrochloride (40 mg, 0.21 mmol) and 1-hydroxy-7-azabenzotriazole (24 mg, 0.18 mmol) were dissolved in 5.0 mL DMF solution. The mixture was stirred for 15 min, then doxorubicin (85 mg, 0.15 mmol) was added and stirred for additional 15 hours at rt. Ethyl ether (50 ml) was added. The precipitate which collected and purified by C18-RP silica chromatography elution with methanol-water to afford desired product **7** as a dark green solid 83 mg (46%). 1H NMR (DMSO-d6, 400 MHz) δ 8.18 (m, 2H), 7.90(m, 2H), 7.62(m, 2H), 7.49(m, 2H), 7.41(m, 2H), 7.32(s, 1H), 7.29(m, 1H), 7.25 (m, 1H), 6.37 (d, 1H), 6.24 (d, 1H), 5.45(s, 1H), 5.18(s, 1H), 4.92(m, 1H), 4.80 (m, 1H), 4.67(m, 1H), 4.52 (m, 2H), 4.21(m, 2H), 4.11(m, 2H), 3.93(s, 3H), 2.96(s, 1H), 2.63 (m, 4H), 2.15 (m, 2H), 2.00 (m, 4H), 1.76 (m, 6H), 1.64(s, 6H), 1.63(s, 6H), 1.56(s, 3H), 1.55(s, 3H),1.48 (m, 2H), 1.20 (m, 2H), 1.08(m, 2H). Mass spectrum (ESI) m/z 1230.47 [M+H]+.

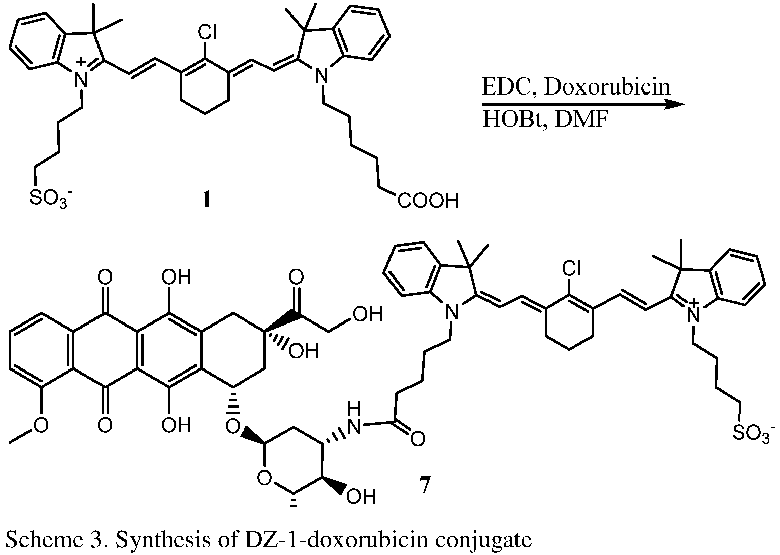

### Reference Example 4.

**Synthesis of DZ-1-artemisinin conjugate 9** (scheme 4): To a solution of dye **1** (250 mg, 0.35 mmol) in methylene chloride (20 mL) was added dihydroartemisinin **8** (110 mg, 0.39 mmol), 1-ethyl-3-(3-dimethyllaminopropyl) carbodiie hydrochloride (82 mg, 0.43) and DMAP (20 mg, 0.16 mmol) and the mixture was stirred 12 hours at room temperature to afford green solution. The solution was filtered and the solvent was removed under reduced pressure. The dye-artemisinin conjugate was purified by C18-RP silica column chromatography elution with methanol-water to afford DZ-1- artemisinin **9** as a dark green solid 179 mg (52%). 1H NMR (DMSO-d6, 400 MHz) δ 8.22 (m, 2H), 7.58(m, 2H), 7.47(d, 1H), 7.38(m, 3H), 7.25 (m, 2H), 6.38 (d, 1H), 6.25 (d, 1H), 5.60(d, 1H), 5.50(s, 1H), 4.55(m, 2H), 4.21 (m, 4H), 2.68 (m, 6H), 2.35 (m, 2H), 2.17(m, 2H), 1.86 (m, 6H), 1.73 (m, 6H), 1.64 (s, 6H), 1.63(s, 6H), 1.57 (m, 2H), 1.37 (m, 2H), 1.24(m, 2H), 1.22(s, 3H), 0.84(m, 3H), 0.67(m, 2H). Mass spectrum (ESI) m/z 971.46 [M+H]+.

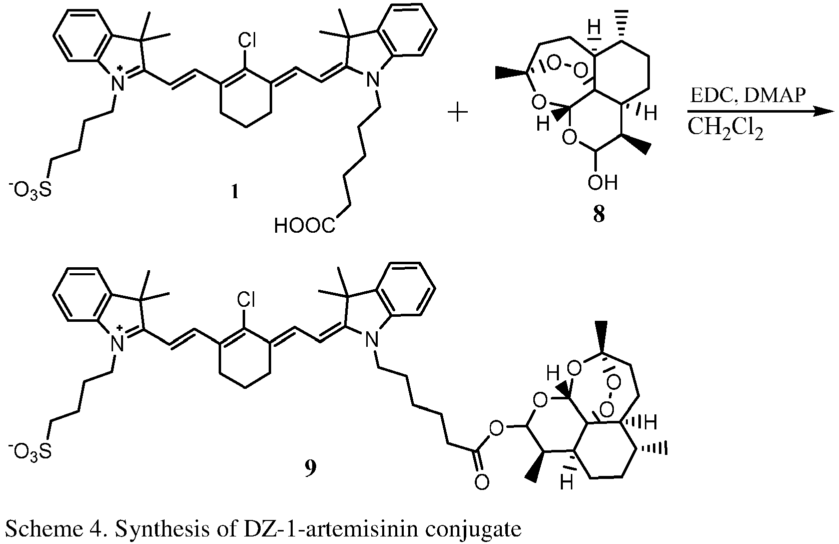

### Reference Example 5.

**Synthesis of DZ-1-puromycin conjugate 11:** The mixture of DZ-1 **1** (130 mg, 0.18 mmol) 1-ethyl-3-(3-dimethyllaminopropyl) carbodiie hydrochloride (52.8 mg, 0.28 mmol) and 1-hydroxy-7-azabenzotriazole (29.8 mg, 0.22 mmol) were dissolved in 5.0 mL DMF solution. The mixture was stirred for 15 min, then puromycin dihydrochloride (340 mg, 0.18 mmol) was added and stirred for 15 hours at rt. Ethyl ether (50 ml) was added. The precipitate was collected and purified by C18-RP silica chromatography elution with methanol-water to afford desired product 11 as a dark green solid 94 mg (44%). Mass spectrum (ESI) m/z 1158.52 [M+H]+.

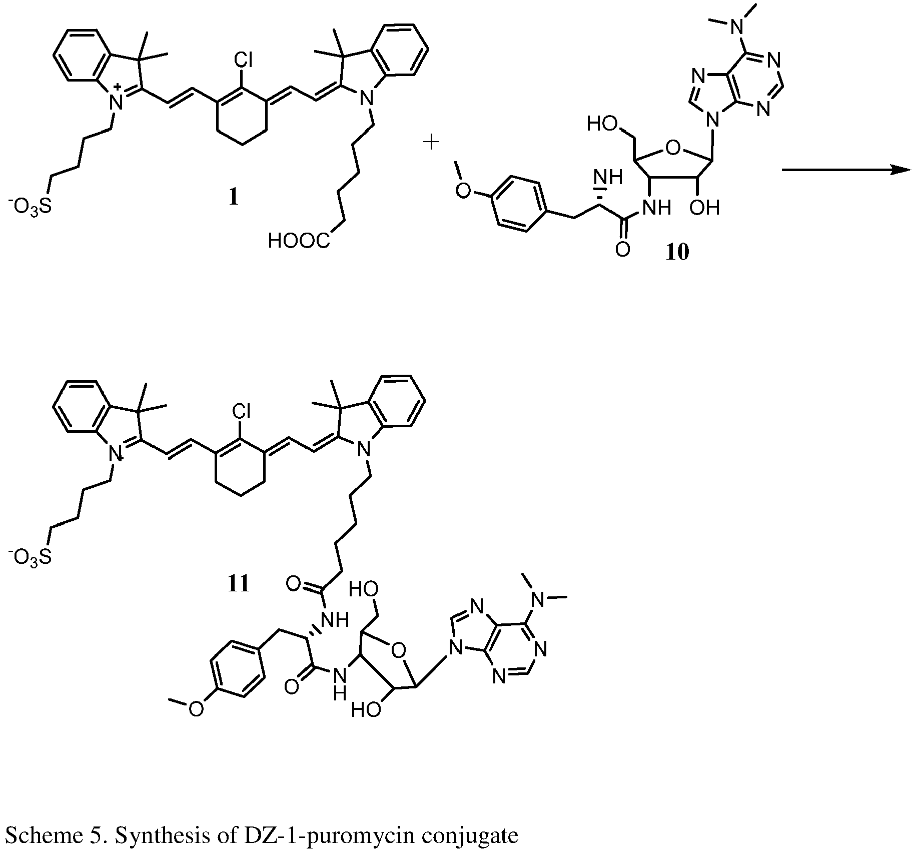

### Example 6.

### Overcoming Renal Cell Carcinoma (RCC) Therapeutic Resistance and Avoiding Renal Toxicity by Conjugating Cisplatin to a Tumor Cell Targeting Near-Infrared (NIR) Carbocyanine Dye

**Background:** Metastatic, recurrent or unresectable clear and non-clear RCC have poor survival despite immunotherapy and molecularly targeted therapies. Clinical trials are the preferred option in patients with metastatic disease (mRCC). Since mRCC is resistant to chemotherapy, we investigated improving cisplatin cytotoxicity toward RCC and reducing its kidney toxicity in mice by conjugating cisplatin to a tumor cell-targeting NIR dye which enters cancer but not normal cells based on tumor cells express high levels of a cluster of organic-anion transporting peptides (OATPs). We compared the cytotoxicity of cisplatin and NIR dye-cisplatin conjugate in both mouse Renca and human RCC tumor models and determined their kidney toxicity in mice.

**Methods:** Growth and viability of immortalized RCC cell lines, Caki-1, o-786, ACHN and SK-RC, human renal epithelial cells and mouse Renca cells were compared upon 72 h exposure to conventional cisplatin and NIR dye-cisplatin conjugate. Apoptosis was assessed by caspase 3/7 luminescent assay. Cleavage of apoptosis substrates was determined by western blotting. NIR dye-cisplatin uptake in cells and tumors was imaged by fluorescent microscopy and IVIS Lumina XR Imaging System. NCr male nude mice or BALB/c male mice bearing respectively s.c. ACHN or Renca tumors were injected i.p. with equimolar doses of NIR dye-cisplatin or cisplatin 3 times a week for 4 weeks. Formalin-fixed tumor, liver and kidney sections were stained with H&E and kidney sections were scored semiquantitativey.

**Results:** All RCC cell lines tested were sensitive to NIR dye-cisplatin but not cisplatin alone with IC50 ranging between 1.94-3.71 µM and >64 µM (p<0.0001), respectively. NIR dye-cisplatin but not cisplain induced apoptosis after 24 hours of drug exposure by luminescent assay of caspase 3/7 and western blot analyses of apoptosis related markers in Caki-1 cells treated with 8, 16, and 32 µM of concentrations of NIR-cisplatin (p<0.0001 to p=0.01). NIR dye-cisplatin injected in mice bearing Renca tumors and ACHN cell line-derived xenografts showed tumor growth inhibiton and apoptotic nuclear lobulation/fragmentation compared to vehicle cisplatin alone (p<0.05). While NIR dye-cisplatin preserved the integrity and normal morphology of mouse kidneys, cisplatin induced marked kidney damages. NIR dye-cisplatin accumulated in tumors 7-15 times above background and was retained for up to 43 days post treatment.

**Conclusions:** NIR dye-cisplatin accumulates exclusively in RCC, and induces RCC tumor apoptosis after 24 hours of drug exposure. Cisplatin alone was completely ineffective against RCC tumor cells and tumor growth, and unlike NIR-cisplatin, showed profound renal toxicity.

### Example 7.

### Three-dimensional prostate cancer circulating tumor cell (CTC) spheroids with chemo- and hormone antagonist resistance in culture can be resensitized by targeted near-infrared dye-drug conjugates.

**Background:** In contrast to most solid tumor cell lines, in vivo and ex vivo prostate cancer (PC) circulating tumor cells (CTCs) exhibit 3D morphology, express stem cell markers and show extreme drug resistance. We investigated the relationship of organic anion transporting peptides (OATPs) and multiple drug resistant gene (ABCB1) expression in PC cells and CTCs grown as 2D versus 3D spheroids. We resensitized drug-resistant PC cells grown as 3-D spheroids with novel near-infrared heptamethine carbocyanine dye-gemcitabine or cisplatin conjugates.

**Methods:** Immortalized PC cell lines (PC3, LNCaP, C4-2, C4-2B, 22Rv1) and 3-D spheroid growth of mouse bone marrow-derived LNCaP and C4-2B and ex vivo expanded CTCs from patients were used. Viability was evaluated by MTT assay and crystal violet staining after 72 hours of drug exposure to cisplatin, gemcitabine, docetaxel, enzalutamide, or abiraterone acetate. Induction of apoptosis was assessed by caspase 3/7 luminescent assay. Growth inhibitory effects were compared between 2-D and 3-D. Senescent luciferase-tagged LNCaP and C4-2B cells from mouse bone marrow regained growth potential when propagated in vitro as 3D spheroids. RPMI medium supplemented with 10% FBS and 1% penicillin/streptomycin was used to grow CTCs in vitro.

**Results:** Immortalized prostate cancer cell lines grown as 2D monolayers are sensitive to docetaxel, gemcitabine, enzalutamide, and abiraterone acetate. In contrast, ex vivo expanded CTCs from PC patients and LNCaP and C4-2B cells from mouse bone marrow grown as 3-D spheroids expressed stem cell markers, Nanog, CD133, c-Myc, FOXM1, FOXM2, CD44, and ALDH1, and showed resistance with no signs of apoptosis after prolonged exposure to docetaxel, abiraterone acetate, and enzalutamide. However, senescent C4-2B cells and CTCs grown as 3-D spheroids in vitro became extremely sensitive to novel NIR dye-gemcitabine and NIR dye-cisplatin conjugates. Uptake and retention of the NIR-dye drug conjugates was partly due to increased expression of OATPs and ABCB1 genes responsible for drug influx and retention in PC cells.

**Conclusions:** Senescent LNCaP and C4-2 cells remained dormant as 3D spheroids in bone marrow but regained proliferative potential when cultured in vitro. Unlike PC cells grown as 2D monolayers, 3D spheroids of ex vivo expanded CTCs are completely resistant to chemo- and hormone antagonists. We show that NIR dye-gemcitabine and NIR dye-cisplatin conjugates resensitize the PC cells grown as 3-D spheroids to chemotherapy.

### Example 8.

Novel Near-Infrared Heptamethine Carbocyanine Fluorescent Dye-Cisplatin Conjugate Demonstrates Significant Antitumor Activity and Overcomes Cisplatin Resistance in MYC-Driven TP53 Mutated Aggressive B-Cell Burkitt's Lymphoma.

**Introduction:** Burkitt's lymphoma (BL) is composed of highly proliferating mature B cells expressing a germinal center phenotype (Cai, Oncotarget 2015). It is one of the fastest growing human tumors and presents in extranodal as well as nodal sites. The genetic hallmark of BL is the MYC translocation with MYC and TP53 mutations in ~60% and 40% of the cases, respectively (Ott, Blood 2014). While prompt administration of multiagent immunochemotherapy regimens is associated with favorable outcomes for the majority of patients, prognosis of refractory or relapsed disease is poor (Jacobson, Blood 2014). Here, we describe the effects of a newly synthesized NIR-dye-cisplatin conjugate (NIR-dye-Cis) on MYC-driven TP53 mutated aggressive B-cell BL cell lines in culture and tumors in mice.

**Methods:** The growth and viability of immortalized B-cell lymphoma cell lines, Namalwa, Raji, CA46, Ramos, Daudi, were evaluated by MTT upon 72 h exposure to conventional cisplatin (Cis) and NIR-dye-Cis. Induction of apoptosis was assessed using a caspase 3/7 luminescent assay. Cleavage of apoptosis substrates and inhibition of c-myc was determined by western blotting, with images quantified by ImageJ after normalization with GAPDH. NIR-dye-Cis uptake in cells and tumors were imaged by NIRF microscopy and IVIS Lumina XR Imaging System, respectively. Male NCr nude and NOD/SCID mice bearing s.c. Namalwa tumors were injected i.p. with equimolar doses of NIR-dye-Cis and conventional Cis 3 times a week for 4 weeks. Formalin-fixed tumor and kidney sections were stained with hematoxylin and esosin and kidney sections scored using a semiquantitative scale designed to assess AKI-associated tubular injury (tubular epithelial cell loss, necrosis, tubular epithelial simplification, intratubular debris, and casts).

**Results:** 1) All BL cell lines tested are sensitive to growth inhibition by NIR-dye-Cis but not Cis with IC₅₀ ranging between 720 nM - 2.53 µM and >64 µM (p<0.0001), respectively. 2) NIR-dye-Cis induces apoptosis after 24 hours of drug exposure as assessed by luminescent assay of caspase 3/7 and western blot analyses of apoptosis related markers (caspase 9, caspase 3 and PARP) in extracts of Namalwa and Raji cell lines treated with 8, 16, 32 µM of concentrations of NIR-dye-Cis with the levels of significance at p=0.0001 to p=0.01; no sign of increased apoptotic activity was observed when these cells were exposed to the conventional Cis. 3) NIR-dye-Cis, but not Cis, downregulates the anti-apoptotic protein c-myc, which is highly expressed in BL cell lines; no significant changes of minimally expressed bcl-2, an antiapoptotic protein, was noted in NIR-dye-Cis and Cis treated BL cell lines. 4) NIR-dye-Cis was found to accumulate 7 to 15 times more in cell line-derived xenografts than mouse kidneys (p<0.0001) and lungs (p<0.0001), and these results were confirmed by NIRF microscopy and IVIS Lumina XR Imaging. 5) NIR-dye-Cis when injected in mice bearing Namalwa cell line-derived xenografts, shows tumor growth inhibition and apoptotic nuclear lobulation/fragmentation when compared to vehicle- or Cis-treated group (p<0.05). 6) Whereas NIR-dye-Cis preserved the integrity and normal morphology of mouse kidneys, by contrast, Cis was observed to induce marked kidney damage as assessed by the histopathology of Cis-treated mice.

**Conclusion:** Aggressive B-cell BL can be treated safely by NIR-dye-Cis. This agent was found to accumulate in BL cells and tumor xenografts and in contrast to Cis, NIR-dye-Cis induced apoptosis and shrinked BL tumor xenografts in mice. NIR-dye-Cis induced apoptosis, downregulated c-myc and overcame Cis-resistance in MYC-driven TP53 mutated aggressive B-cell BL. This agent could have potential clinical benefit in relapsed/refractory heavily pretreated patients for improved efficacy and reduced kidney toxicity. NIR-dye-Cis could be employed as the secondary agent for the treatment of relapsed/refractory aggressive B-cell non Hodgkin lymphomas.

### Example 9.

**Introduction:** To understand the possible direct effect of statins on prostate cancer (PC) cells, we embarked on studies in which we synthesized a simvastatin (SM)-NIR dye conjugate [i.e. SM chemically conjugated to a tumor-targeting near-infrared (NIR) heptamethine carbocyanine organic dye, referred to in this application as NIR-SM] that can target PC cells directly, bypassing the liver. We found NIR-SM to be superior to SM in inhibiting the growth of PC cells in culture. Moreover, NIR-SM, but not SM, was also found to sensitize PC cells toward cytotoxic effects of the currently commonly used agents in the clinic for the treatment of castration-resistant prostate cancer (CRPC) and its metastatic form, mCRPC: abiraterone acetate (AA), enzalutamide (EZ) and docetaxel (DT).

**Methods:** 1) determine why NIR-SM but not SM can re-sensitive PC tumors toward growth inhibition by AA, EZ or DT, particularly when PC cells are cultured as 3D spheroids; and 2) to assess the underlying molecular mechanisms of how NIR-SM re-sensitizes therapeutic effects of AA, EZ or DT in human PC. Our *approaches* emphasize the effects of NIR-SM on: 1) the growth of tumor cells as 3D spheroids and as tumor xenografts, 2) the uptake, retention and metabolism of NIR-SM and SM in AA, EZ or DT treated cultured PC cells, as 2D monolayers vs 3D spheroids, 3) the comparative aspects of NIR-SM and SM on cell adhesion, growth, survival, EMT and metastasis, 4) using computational approaches to predict or monitor the effectiveness of NIR-SM in re-sensitization of PC cells toward therapeutic effects exerted by AA, EZ or DT. Since our ultimate goal is to develop NIR-SM as an effective agent to re-sensitize PC patients who fail CRPC therapy, we initiate a new approach of creating an orally bioavailable NIR-Linker-SM which could fit into the active pocket of the HMGCR enzyme.

We synthesize, characterize and assess the biologic activity of NIR-SM conjugates on PC cells and tumors in the presence or absence of DT, AA or EZ. Without being bound by theory we hypothesize that NIR-SM conjugates are more effective and less toxic than SM in overcoming therapeutic resistance developed in PC cells and tumor xenografts in hosts toward androgen antagonists or chemotherapy with AA, EZ or DT, respectively. We test this hypothesis by the following studies:

Furthermore we also wanted to examine why NIR-SM, but not SM, can enhance PC cell growth inhibition in 3-D spheroids, and why NIR-SM is superior to SM in re-sensitizing PC cell growth toward AA, EZ, or DT in vitro: effects of OATPs and ABC transporters.

**Results:** We used a series of commonly used human PC cell lines and found no obvious difference in IC50 between SM and NIR-SM, ranges from 1 to 7 µM, with differences dependent upon the cell types used for the assay. We used a low concentration of NIR-SM, 0.25 µM, which has marginal effects on the growth of PC cells, but it sensitizes the cytotoxic effects of AA and EZ in both control and AA/EZ resistant PC cells in the C4-2B background 4-5 fold (**Table 1**).

**Table 1. NIR-SM sensitizes AA and EZ naïve and resistant C4-2B PC cells in vitro cultured as 2D monolayer**

| **Cell type** | **NIR-SM (µM)** | **EZ IC50 (µM)** | **Cell type** | **NIR-SM (µM)** | **AA IC50 (µM)** |
|---|---|---|---|---|---|
| **Naive C4-2B** | **0** | **7.69** | **Naive C4-2B** | **0** | **6.79** |
| | **0.25** | **1.50** | | **0.25** | **1.65** |
| **Resistant C4-28** | **0** | **10.69** | **Resistant C4-2B** | **0** | **13.31** |
| | **0.25** | **1.56** | | **0.25** | **3.32** |

Using *ex vivo* propagated CTCs from human PC patients, we found IC50 of NIR-SM is about 4-fold lower than SM, at 5 and 20 µM, respectively. Most dramatically, we found when PC cells were exposed to equal molar concentration of these statins, NIR-SM but not SM dramatically enhanced the cytotoxicity exerted by AA, EZ or DT, three of the most commonly used drugs for the management of CRPC and mCRPC patients, by 21- to 122-fold (**Figure 1**).

We confirmed the role of OATP in mediating NIR-drug conjugate uptake in PC cells by documenting that NIR-drug conjugate uptake and retention in cancer cells can be completely abolished by either BSP, a competitive inhibitor of OATP carriers or by genetic knockdown of specific isoforms of OATPs drug carriers.

Additionally, we conducted studies where NIR dye, SM, and NIR-SM were identified in PC cells by liquid chromatography mass spectrometry (LC-MS). HPLC retention times for NIR, SM, and NIR-SM are 25.5, 27.5, and 35 minutes, respectively. Respective molecular weights of these compounds in cells are 705, 419 and 1,105 daltons, as determined by MS. By following UV absorption at 782 nm for NIR-SM signal, we derived a standard curve with linearity of absorbance extending from 1 to 14 µM (r=0.99) and found that NIR-SM accumulates in PC cells an average of 5-fold higher in PC 3D spheroids than those of PC 2D monolayer.

***Methods:*** Without being bound by theory PC cells grown as 3D spheroids is a preferred model for NIR-SM studies for the following reasons: 1) *ex vivo* propagated CTCs grown as 3D spheroids, expressed sternness, NE and EMT genes, which are well-represented in aggressive and drug-resistant PC cells. We observed CTCs cultured as 3D spheroids, exhibiting invasive, migratory phenotype in culture and metastasis in mice; 2) PC cells with a stem cell phenotype, could have initiating roles in cancer growth, metastasis and resistant to therapy, and are an important cell population to target. These cells are often resistant to hormone and chemotherapy; 3) we observed PC cells grown as 3D spheroids exhibit most profound difference in their therapeutic responses and re-sensitization by NIR-SM when compared to SM, with CTCs grown as 3D spheroids, challenged by AA, EZ or DT *in vitro* (**Figure 1**)*;* 4) Therapeutic resistance of PC toward hormones and chemotherapies could be recapitulated by 3D spheroid growth of PC cells. Because CTCs cannot grow as a 2D monolayer, we have established a CRPC cell line, C4-2B, grown both in 3D spheroids and 2D monolayer, with cells grown in basal medium (2D), switched to medium enriched with bFGF and EGF (3D, data not shown). We have authenticated this cell line and also patient-derived CTCs. We also have further conducted characterization studies in which the chromosomal G-banding of the cell lines are determined using conventional cytogenetic approaches. Without being bound by theory, we found that, compared to CTCs, the used of C4-2B cells under 2D and 3D culture conditions may provide a better comparison with other PC cell lines, which are commonly grown as 2D monolayer. CTCs/C4-B are cultured and exposed to either SM or NIR-SM (**Figure 2****,** chemical structures of compounds 1 and 2) for one hour. Uptake and retention of NIR-SM and SM and their metabolites are determined by LC-MS to confirm the chemical structures of the parent compounds and their metabolites in PC cells. PC cells are exposed to NIR-SM and SM for a period of 15 min (uptake) to 2 hrs (retention) and the parent compounds and their metabolites in cells are quantitatively analyzed after extraction with acetonitrile and water (hydrophilic fraction), then with chloroform: methanol (3:1 v/v) (hydrophobic fraction). Finally, the pellets are sonicated, centrifuged, and evaporated for analysis of the macromolecule bound fraction by MS. Chemical analyses are conducted using standardized protocols.

To determine the influx/efflux role of OATPs and ABC transporters in CTCs/C4-2B, we perform genetic knockdown of selected OATPs, OATP1B3, OATP1A4, and ABC transporter 1B1, in CTCs/C4-B models. In separate experiments, we also expose cells to an OATP competitive inhibitor, BSP, and/or ABC transporter inhibitors that interfere with membrane lipids, and phenothiazines, to determine the role of OATP and ABC transporter membrane carriers that will quantify influx and efflux of NIR-SM and SM. We also analyze the presence of AA, EZ or DT, and their metabolites by LC-MS in PC cells exposed to NIR-SM or SM. These studies allow us to assess if NIR-SM or SM may compete with AA, EZ or DT for OATP or ABC transporter carriers for influx and retention into PC cells. Alternatively, NIR-SM or SM may lower membrane cholesterol level, alter membrane carrier distribution and avidity for drug transport to enhance AA, EZ or DT uptake and retention in cells. We correlate the drug concentration within the cells with their growth inhibitory in PC cells to determine if NIR-SM re-sensitization of AA, EZ or DT can be explained by increased overall drug uptake and retention in cells.

Without being bound by theory, we hypothesize increased uptake of NIR-SM in comparison to SM in 3D CTC spheroids. This increased uptake may be explained by elevated OATPs and ABC transporters in 3D CTC spheroids. Without being bound by theory, we hypothesize that these membrane carriers are important for the transport and retention of NIR-SM and SM in PC cells. Without being bound by theory, by interfering with either OATP or ABC transporters with BSP or phenothiazines, respectively, or by silencing approaches, we hypothesize that uptake of NIR-SM and SM will be suppressed. Without being bound by theory, we hypothesize that significantly higher levels of NIR-SM than SM retention in 3D CTCs/C4-2B spheroids than that of the 2D C4-2B conditions, possibly due to higher levels of expression of OATPs and ABC transporters arising from hypoxia in 3D conditions. Alternatively, without being bound by theory increased NIR-SM but not SM accumulation in 3D CTCs/C4-2B spheroids could be explained by structural features of NIR-SM because once NIR-SM enters into PC cells, it forms a tight complex non-covalently with nucleic acids and proteins intracellularly. Without being bound by theory, we hypothesize parallel increases in AA, EZ or DT in cells treated with NIR-SM, but to a lesser extent in SM-treated cells and in 2D, possibly contributed by elevated OATPs and ABC transporters. Without being bound by theory, we hypothesize drug concentration alone cannot explain the re-sensitization observed, but rather the combined effects of drug accumulation in cells plus the direct action of SM intracellularly on cell growth, survival and apoptosis mechanisms, could account for the re-sensitization of PC cells toward AA. EZ or DT.

### Example 10

**Introduction:** Our goal was to compare the effectiveness of NIR-SM and SM in re-sensitizing PC xenografts growth in mice, treated with AA, EZ or DT. Unlike SM, which distributes randomly in body fluids and acts on host liver to lower serum cholesterol, NIR-SM, in contrast with its tumor-targeting ligand, is retained preferentially in tumors and exerts its cytotoxic effects locally on tumor cells. Because of the differential effect of NIR-SM but not SM in re-sensitizing the growth inhibition of drug-resistant tumor cells toward AA, EZ or DT, in this study we test if similar effects are seen in PC tumor xenografts in mice. This study collects data from a head-to-head comparison of SM and NIR-SM in enhancing the therapeutic effects of AA, EZ or DT, in PC tumor models in mice.

**Methods:** We have established 11 *ex vivo* propagated CTCs from PC patients. These cell lines are robust in their ability to form tumors at s.c., orthotopic and intraosseous sites, with an efficiency ranging from 50-100%. A representative CTC-derived s.c. xenograft (abbreviated CDX), stained positively for PC markers AR, PSA and PSMA (**Figure 3**). We genetically tagged one of the CTCs with luciferase, and when injected orthotopically, it metastasized to distant organs. We profiled the gene expression of CTCs, and compared them against indolent PC cells, such as LNCaP, we found these cells expressed genes commonly associated with an aggressive phenotype, the metastasis-initiating cells (MIC). Here is a protein profile of two CTC clones from two different patients (**Figure 4**). When compared to an indolent line, DC-1 (established from a PC patient with localized disease) or LNCaP cells, CTC clones were found to express genes, detected by western blot, commonly associated with aggressive oncogenic functions, such as epithelial-to-mesenchymal transition (EMT), sternness and neuroendocrine (NE). These CTC and CDX models are used to test the effectiveness of NIR-SM, which without being bound by theory we hypothesize exerts its actions directly on PC tumors. **Figure 5** shows NIR-SM stained PC cells in culture, grown either as 2D monolayer (PC-3 cells) or as 3D CTCs spheroids. NIR-SM was found to reside *in vivo* exclusively in tumors, as detected by fluorescence imaging of subcutaneous PC-3 tumors in mice.

Luciferase-tagged CTCs and C4-2B are used in this *in vivo* study. These cells were characterized as 3D spheroids *in vitro,* are responsive *in vitro* toward NIR-SM by lowering significantly their IC50 toward cytotoxic effects exerted by AA, EZ or DT. They also form robust solitary xenograft tumors, or the CDXs, at the site of tumor cell inoculation. To conserve the number of mice used in this study, we use a two-step approach: First, we conduct a pilot study to address the question of whether subcutaneous PC tumors respond to sensitization by NIR-SM on current PC therapeutic regimens, AA and EZ or DT. Four groups of male athymic nude mice (Charles River, Inc) between 6 to 8 weeks of age (48 mice/group, with each mouse inoculated with two tumors/mouse), receive s.c. tumor cell inoculation (1x106 CTCs). In a pancreatic cancer transgenic KrasLSL.G12D/+; p53R172H/+; PdxCretg/+ (or KPC) model, we detected a tumor with fluorescence imaging of NIR-SM, estimated to be 1.1 mm (**Figure 6**). Mice are randomized to receive daily administration of either vehicle, AA (15 mg/kg, x5), EZ (10 mg/kg, x5) or DT (15 mg/kg, x3) (12 mice/group). Among these treatment randomizations, mice are assigned to two groups, with half of the mice (6 mice or 12 tumors per group) to receive vehicle and the other half receive daily NIR-SM (8 mg/kg, x2) to test if NIR-SM is better than vehicle in sensitizing tumor regression exerted by AA, EZ or DT. We monitor tumor sizes by bioluminescence imaging every two weeks with tumor sizes also measured by a caliper weekly. At the end of a 12-wk observation period, mice are sacrificed and tumors are harvested and subjected to routine H/E and IHC analyses. Special attention is directed to cell proliferation (Ki67), apoptosis (TUNEL, caspases), angiogenesis (CD34), and expression of tumor markers relevant to the aggressiveness of the tumors and markers are indicative of PC (AR, PSMA, PSA).

Without being bound by theory, we hypothesize NIR-SM to be more effective than SM in inhibiting the growth of CDX in mice. Without being bound by theory we hypothesize that NIR-SM enhances further the regression of CDX tumors grown at either s.c. or i.t. sites by all therapeutic agents tested. Without being bound by theory there could be differences in re-sensitization dependent on the therapeutic agents, and if this difference found *in vitro* is replicated in mice, we hypothesize NIR-SM will be most effective in reversing resistance with greater efficacy toward DT, followed by AA, and is less efficacious in sensitizing EZ. Without being bound by theory we hypothesize the trend of enhanced tumor-regression induced by NIR-SM to be the same in CDXs and C4-2B tumors, provided if the C4-2B tumor line we used for inoculation is maintained as a 3D spheroid growth *in vitro.* Without being bound by theory we hypothesize that it is possible NIR-SM sensitizes specifically PC grown as 3D spheroids, but not as 2D monolayer due to the expression of OATPs and ABC transporters, which could be regulated by hypoxia. 3D spheroids would be more relevant in this context than 2D monolayer cultures.

Without being bound by theory we hypothesize tumors harvested from mice treated with combined NIR-SM + AA, EZ or DT (but not SM) will have increased expression of apoptotic genes, and decreased expression of proliferation, survival and angiogenesis genes and biomarkers. Without being bound by theory we also hypothesize NIR-SM to reduce the expression of aggressiveness-related genes in PC cells. Without being bound by theory, we hypothesize that in comparison to vehicle controls, there will be a reversal of PC phenotype from the treated tumors in which the PC cells within the tumors may undergo a reversal of EMT (known as MET), with decreased expression of sternness, and NE biomarkers.

*Ex vivo* propagated CTCs and their CDXs are new tumor models and their behavior needs to be carefully monitored and documented. For example, we noted some of the CTCs did not express AR and AR targeted genes in culture, but gained the expression of AR and AR targeted gene in CTCs-derived CDXs.

### Example 11

**Introduction:** Our goal was to develop an orally bioavailable NIR-Linker-SM that will fit into the pocket of HMGCR enzyme and to conduct a preclinical study using PC cell and animal models. The challenge we need to address is whether we can develop an orally bioavailable alternative that can be used to re-sensitize AA, EZ or DT therapy for PC patients. We examined the crystal structure of the statin target, HMGCR, and based on this structure, we designed a stable and potentially orally bioavailable SM conjugate, NIR-Linker-SM **(****Figure 2****,** compound 3 without linkers and compound 4 with linker) that is synthesized, characterized and tested in the model systems described above.

We have synthesized and characterize two new SM conjugates based on the crystal structures of HMGCR (**Figure 7**). We compare the biologic activities of NIR-Linker-SM either with or without linker with the tumor-targeting NIR dye. The active SM is attached to NIR dye through an ether rather than an ester linkage with a variable length and hydrophilicity of linkers comprised of either linear saturated hydrocarbon chains (-(CH2)n-, n = 3-8) or polyethylene glycols (-(CH2CH2O)n-, n =2, 4, 6, 8, 12). The identity of these conjugates is confirmed by LC-MS and their acid stability preparing for oral delivery is tested using routine protocols.

**Methods:** We investigate the biologic activity of the chemically synthesized and LC-MS characterized NIR-Linker-SM, with variable linkers as described above. We characterize their biologic activities on 3D CTCs spheroids. Based on their activities in synergizing growth inhibitory effects of AA, EZ or DT, we select the best possible conjugate for additional *in vitro* and *in vivo* studies. Key studies are: 1) To insure the biologic active NIR-Linker-SM can be detect in PC cells without evidence of enzymatic degradation; 2) To compare the IC50 of the NIR-Linker-SM with the NIR-SM in both 2D and 3D cultured CTCs/C4-2B; 3) To determine acid stability of NIR-Linker-SM and NIR-SM *in vitro* and to assure the bioavailability if it is given by oral route; 4) To determine whether it is true that NIR-Linker-SM but not intact or hydrolyzed NIR-SM can be detected in PC tumors and CDXs in mice.

Without being bound by theory, based on the crystal structure of HMGCR, we hypothesize NIR-SM with linkers will work better than the ones without the linkers. Without being bound by theory, we hypothesize NIR-Linker-SM to be acid stable. Without being bound by theory, when given orally, we hypothesize NIR-Linker-SM but not SM can be detected in cells and tumors as an intact molecule. These results support the notion that as long as SM can fit into the active center of HMGCR, the new NIR-Linker-SM reaches the target when delivered orally and exerts its activity as an intact conjugate without the need of enzymatic release of SM locally in the tumor cells.

While we have the ability to synthesize and characterize the new NIR-Linker-SM, the activity of inhibiting HMGCR directly by this conjugate without enzymatic digestion can only be tested and confirmed biologically in cells. We apply additional computational models to synthesize additional NIR-Linker-SM using different linkers with different chemical properties. We choose peptide based linker for further synthetic modification to optimize the pharmacokinetic parameters of the NIR-SM conjugate. Molecular modeling is employed to aid in the design and evaluation of the peptide linkage. The linker module includes cleavable and non-cleavable peptide sequences with single or multiple amino acids to optimize the drug binding, solubility and clearance properties.

### Example 12

**Introduction:** Our goal is to determine the molecular mechanisms by which NIR-SM conjugate re-sensitizes the growth inhibition by AA, EZ or DT in PC cell lines and *in vivo* tumor models. Without being bound by theory, we hypothesize that the NIR-SM conjugate overcomes therapeutic resistance through increased uptake and accumulation of SM and appears to act in concert with AA and EZ to interrupt AR signaling, and with DT to block microtubular assembly in mitotic and interphase cellular signaling. In addition, AA, EZ or DT inhibition is synergized by direct SM action in blocking small G proteins (like Ras, Rho and Rac) isoprenylation and lipid raft-mediated activation of PI3K-Akt in PC cells. NIR-SM re-sensitization of AA, EZ or DT therapy can be detected with a *cholesterol signature* that has predictive value in assessing PC virulence and NIR-SM responsiveness.

### Example 13

**Introduction:** Our goal is to determine if NIR-SM is more effective than SM in blocking AR-and Akt-mediated signaling and microtubular dynamics in PC cells, in inhibiting small G proteins, Ras, Rho and Rac isoprenylation. A large number of SM targets have been identified by lowering cholesterol biosynthesis from host liver. Here we validate if NIR-SM is more effective than SM in enhancing AA and EZ in blocking AR signaling pathways and in antagonizing DT-induced microtubular signaling network. We confirm the direct action of NIR-SM on G protein prenylation and PI3K-Akt activation of the lipid raft microdomains and ultimately alter the growth, survival, invasion and metastasis of PC cells, 3D CTCs spheroids and CDXs. Previous work from the Freeman lab has shown that a hypercholesterolemic diet increased PC tumor growth and that lowering serum cholesterol can reduce PC growth, reduce PI3K-Akt pathway activity and inhibit tumor angiogenesis. We have extended these observations by demonstrating that hypercholesterolemic mice show increased incidence and earlier onset of PC bone metastasis and elevated numbers of CTCs (**Figure 8**). In the past year, Chung and Freeman have investigated if SM effects on PC cells can be predicted using gene expression signatures that reflect cholesterol homeostasis and statin responsiveness. Drs. You and Freeman have recently described a novel PC subtyping system based on an algorithm developed using an unprecedentedly large transcriptome dataset. Three subtypes identified in this study show distinct gene ontology profiles that imply differential sensitivity toward manipulation of cholesterol metabolism. In additional experiments we define a gene expression signature that reports sensitivity to NIR-SM and we test this signature for its predictive value using animal models and clinical specimens.

**Methods:** To examine if AR signaling may be more effectively perturbed by NIR-SM plus AA or EZ than SM plus AA or EZ, we use well-characterized ARE-reporter systems, such as PSA-luciferase (PSA-Luc) stably transduced C4-2B 3-D spheroids as a model for the assessment of AR transcriptional activity. Positive and negative controls of this model are measured in cells treated by either R1881 or vehicle, respectively. PSA-Luc is measured in stable PSA-luc expressed C4-2B 3-D spheroids, treated with AA or EZ in the presence or absence of NIR-SM or SM.

Microtubule dynamics are measured in NIR-SM or SM treated PC cells in the presence or absence of DT according to published protocols. Microtubule dynamics are assessed by fluorescence imaging methods, where immunolabeled tubulin is the readout for measurements of polarized forced generation, contractility and response to substrate stiffness measured in the presence of DT using established protocols. We have shown that DT enhances microtubule polarization and inhibits shrinkage of microtubule length. We test other agents, such as nocodazole and vincristine, which without being bound by theory we hypothesize block the polymerization of tubulin into microtubules, and cold temperature that can cause a rapid depolymerization of microtubules. With these controls, we determine if NIR-SM or SM may differ in enhancing the activity of DT in promoting microtubule polymerization in PC cells.

To determine if protein prenylation may be affected differentially by NIR-SM plus AA, EZ or DT vs. SM plus AA, EZ or DT, we use a non-radioactive liquid chromatography tandem mass spectrometry (LC-MS/MS) method. Protein prenylation is assayed by bioorthogonal labeling of prenyl-proteins with fluorescent or affinity tags, using a Cu-catalyzed alkyne azide cyclo-addition (CuAAC) reaction. Briefly, cells are pre-exposed to metabolic labeling with C15Alk-OPP, an isoprenoid analog. Labeled cells are permeabilized and then reacted chemically with a fluorescence probe, 5-Fam-PEG-N3, or an affinity tag, az-azo-biotin, through CuAAC reaction. For imaging and quantification of the prenylome, fluorescence tag-labeled cells are analyzed by confocal microscopy and flow cytometry. For protein-specific quantification of prenylation levels, affinity tag-labeled proteins are specifically enriched by streptavidin affinity purification, followed by protein digestion and LC-MS/MS. Subsequently, label-free quantification is performed to determine the relative prenylation levels of >100 prenylated proteins across the different conditions.

To determine if NIR-SM is more effective than SM in inhibiting PI3K-Akt phosphorylation in lipid rafts fractions and induction of apoptosis in PC cells, we use our previously established methods to conduct these assays in cell-free extracts for PI3K-Akt phosphorylation and kinase activity, and for apoptosis using DNA fragmentation, TUNEL, and caspase activity assays.

Without being bound by theory, we hypothesize NIR-SM to be superior to SM in growth inhibition and apoptosis activity, reducing AR-mediated transcriptional activity, tubulin polymerization, and prenylation of small G proteins such as Ras, Rho and Rac, and PI3K-Akt activity in the cells and in lipid raft fractions. Without being bound by theory, we hypothesize these broad-range of activities to be further enhanced by the co-administration of AA, EZ or DT. To determine if these altered cellular functions by NIR-SM and SM either with or without AA, EZ or DT is specific due to the lowering of cell membrane cholesterol, we investigate if exogenously added cholesterol or 4-mevalonate can reverse the alterations of these cellular functions.

### Example 14

**Introduction:** Our goal is to establish a cholesterol-sensitivity signature using NIR-SM and SM in cultured 3-D CTCs/C4-2B spheroids and in CDX/C4-2B. Assess the relationship between cholesterol sensitivity to other processes, including androgen response and microtubular dynamic signatures. Test the degree to which the cholesterol-sensitivity signature is relevant to PC patients. Gene expression profiles are combined with computational approaches to address the mechanism of differential action of NIR-SM and SM that we have observed in our preliminary studies. The Freeman group recently used a large collection of human PC transcriptome profiles and a novel algorithm, based on pathway activation signatures of known disease relevance, to develop and validate a novel subtyping system for PC. They showed that PCs can by classified into as few as three types, designated PCS1, PCS2 and PCS3, which exhibit luminal (PCS1 and PCS2) and basal (PCS3) phenotypes. PCS1 exhibits poor prognosis, and is more likely to progress to metastatic disease, including in low Gleason sum tumors. This study shows our ability to use integrated analysis of gene expression patterns to reveal novel PC biology with clinical relevance. Here we take a similar approach by performing RNA expression profiling of NIR-SM and SM treated 3-D CTC/C4-2B spheroids and tumor implants in mice. From this large dataset, we develop a signature that reports sensitivity to cholesterol manipulation. We test whether this signature has utility in predicting disease progression in PC patients.

**Methods:** In order to define a cholesterol-sensitivity signature, we first produce global gene expression profiles by profiling the 3-D spheroids and xenograft tumors following treatment with NIR-SM and SM (and appropriate controls) using RNA sequencing (RNA-seq). The quality of sequence reads are assessed and low quality reads are filtered using the FastQC tool (Babraham Bioinformatics, Cambridge, UK) and ShortRead (version 1.30.0) package from R bioconductor (version 3.3). To determine expression levels of transcripts, we quantify and summarize them through the use of Kallisto and tximport R software package with the UCSC hg38 build of the (Homo sapiens) genome. To reduce systemic bias between samples, we apply trimmed mean of M-values (TMM) normalization method to gene expression data. Given the normalized expression datasets, we identify differentially expressed genes (DEGs) by comparing NIR-SM, SM and control treatment conditions. We use criteria with false discovery rate (FDR) < 0.05 and fold change ≥ 2 using a method based on negative binomial distribution. DEGs from the treatment with NIR-SM represent cholesterol-sensitive genes and DEGs from the SM treatment represent cholesterol-insensitive genes. We then define a Cholesterol Sensitivity (CS) signature by subtracting cholesterol-insensitive genes in SM treatment from cholesterol-sensitive genes in NIR-SM treatment. The subtracting step allows us to refine the cholesterol sensitivity signature by getting rid of SM driven gene expression changes that are irrelevant to NIR-SM dependent gene expression changes. We validate the gene and protein expression of the subset of genes in the CS signature through qRT-PCR and western blot analysis with the 3D spheroid cultures.

Next we assess relationship between the CS signature and other signatures we have retrieved (and sometimes refined) from the literature and/or public or proprietary databases, = including AR responsiveness, microtubule dynamics, and others that we assign as relevant to this project. Expression levels of the three signatures are extracted with the gene expression profiles of 1,321 PC patients that we have assembled and described. We compute the relationship of the CS signature to the other disease-relevant signatures, and to the subtype assignments we have previously made in this large cohort using the Z score method. Correlation patterns are visualized and inspected with scatterplots and regression curves (**Figure 9**).

To determine whether the CS signature predicts "cholesterol sensitivity" or "cholesterol resistance" in human PC, we ask whether a classifier based on the CS signature can segregate PC patients into two classes using a naive Bayesian algorithm applied to the PC cohort above (1,321 patients). The classifier accuracy is assessed with 10-fold cross-validation method and ROC curve analysis on the same cohort. In addition, we analyze 8 human PC cell lines from The Cancer Cell Line Encyclopedia (CCLE) and 11 PC mouse models and assess whether any correlation of pathological characteristics with classification result in order to determine whether the CS signature derived classification is relevant to pre-clinical models of PC.

To determine the prognostic relevance of the CS signature, we investigate how much the classifier from the CS signature improves the prediction of clinical outcomes in the Decipher GRIDTM cohorts. Specifically, we assess the ability of the CS signature to predict the risk of clinical progression after ADT therapy using the Cox proportional hazard models of tumor grade, rate of metastasis, and PC specific mortality (PCSM). Hazard models of CS signature associated with a significant risk of progression are confirmed by ROC analysis with 10-fold cross validation method. We also present area under the survival ROC curve for direct comparability to the Decipher GRIDTM cohorts. In addition to assessing predictive power of prognosis after ADT responsiveness, we assess the responsiveness to EZ in CTCs of CRPC patients. For this purpose, we perform the unsupervised clustering analysis to investigate expression pattern of genes in the CS signature in the CTC dataset, which contains single cell RNA-seq data from 77 intact CTCs isolated from 13 patients. We apply the CS signature derived classification scheme to cluster the 77 CTCs by the hierarchical method. Groups are assessed as to whether CTC group with high and low probability from the classifier derived from the CS signature represents distinct response to antiandrogen by the odds ratio test with response information.

Without being bound by theory these experiments provide novel insights into mechanisms that provoke growth inhibition and apoptosis in PC cells when HMGCR is inhibited. They reveal the differential biological effects of NIR-SM vs. SM, and provide a thorough analytic framework to test the potency and specificity of the NIR-Linker-SM agent.

### Example 15

Improved positron emission tomography (PET) imaging on a tumor is performed using DZ1-18FDG or DZ1-19FDG, results for DZ1-19FDG are shown in **Figure 38****.** The figure shows human lung tumors in mice exposed to DZ1-19FDG (right panel), compared to an imaging agent that incorporates 64Cu (DZ-1-DOTA), shown on the left panel, which shows substantial accumulation of dissociated 64Cu in mouse liver. 19FDG (and similarly 18FDG) shows improved PET imaging of tumors and avoids undesirable accumulation in the liver. Fluorescence images (here, for example, of a human lung tumor signal from a xenograft in mice) could be obtained at 3.5 hrs with stable isotope 19F used instead of 18F to avoid unnecessary radiation (both resulting FDG compounds or conjugates thereof are equivalent for the purpose, with exception of lack of radiation). The 19F results thus demonstrate the clinical feasibility of the imaging agent, which advantageously allows imaging tumors using 18FDG after a short time compared to known imaging agents, i.e. less than about 4 hrs after injection thereof (based on the known 18F T1/2=110 min). Often the alternatives take much longer, e.g. 48 hours shown the image acquired on the left panel for 64Cu-tagged DZ-1-DOTA. Because of their similarity and covalent bonding of 18F or 19F to the DZ1-conjugate, both DZ1-18FDG or DZ1-19FDG equally have this advantage in time reduction over 64-Cu tagged DOTA, further they both eliminate demetalation of the radionuclide to the liver (avoiding liver toxicity), and at the same time provide good imaging signals within a desirable time frame. Thus DZ-1-18FDG may serve to greatly reduce the time after which PET imaging can be performed in patients, which may be possible within less than a day, less than 8 hours, less than 4 hrs, less than 2 hours, less than 1 hour, or even sooner, after injecting DZ-1-18FDG as the imaging probe.

Synthesis of imaging agents: The imaging agents may be synthesized as described below. DZ-1-18FDG or DZ-1-19FDG is formed as shown below for glucose. 18FDG is also known as Fludeoxyglucose (18F), fludeoxyglucose F 18,fluorodeoxyglucose, or abbreviated as [18F]FDG,18F-FDG). 19FDG similarly is fludeoxyglucose F 19.

DZ1-18FDG conjugate is synthesized as shown in reaction scheme 6 below, DZ1-19FDG conjugate is similarly synthesized, using the respective FDG solution.

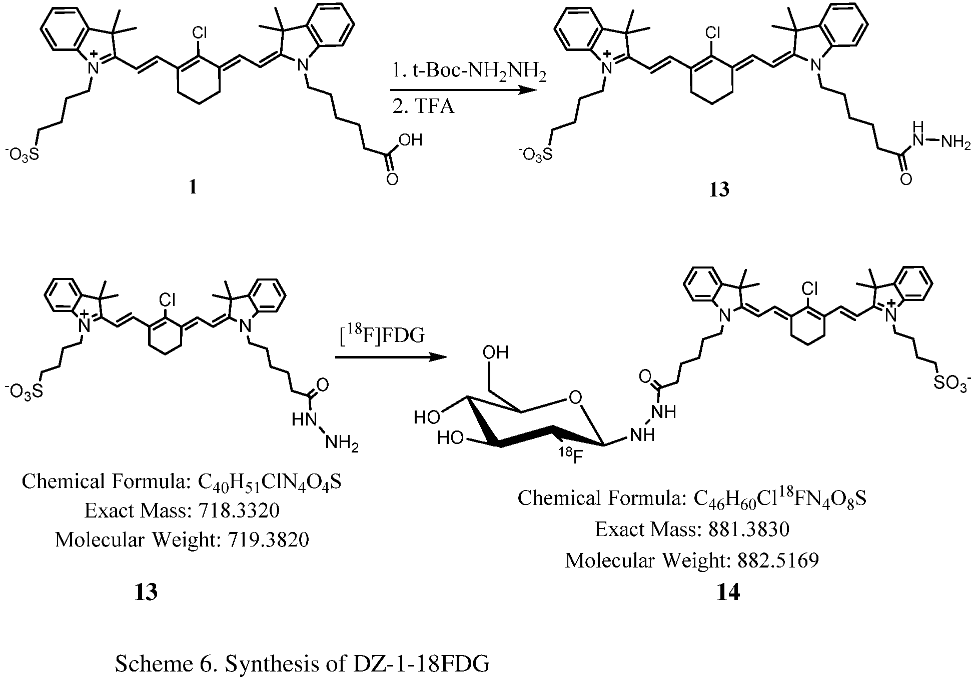

The mixture of DZ-1 **1** (100 mg, 0.14 mmol) 1-ethyl-3-(3-dimethyllaminopropyl) carbodiie hydrochloride (40 mg, 0.21 mmol) and 1-hydroxy-7-azabenzotriazole (23 mg, 0.17 mmol) were dissolved in 5.0 ml CH₂Cl₂ solution. The mixture was stirred for 15 min, then t-boc-hydrazide (19 mg, 0.14 mmol) was added and stirred for additional 2 hours at rt. The solvent was removed under reduced pressure and the product was purified by C18-RP silica chromatography elution with methanol-water to afford DZ-1-boc-hydrazide as a dark green solid. The product was dissolved in 20% TFA in CH₂Cl₂ (5ml) for 2h, and 40ml of ether was added. The precipitate was collected washed with ether and dried under vacuum to give the desired hydrazide product **13**, 31 mg (31%). Mass spectrum (ESI) m/z 719.33 [M+H]⁺.

Dissolve DZ1-hydrazide **13** (100µg) in 0.2 ml of 30% glacial acetic acid in Methanol (v/v). The solution is added to FDG solution. The mixture is incubated at 60°C for 30min, and the product was purified by RP HPLC. Analytical reversed-phase high-performance liquid chromatography (RP-HPLC) is performed with a C18 reversed-phase column (5 µ, 150 × 4.6 mm). The mobile phase is changed from 60% Solvent A (0.1% TFA in water) and 40% Solvent B 0.1% trifluoroacetic acid in 80% aqueous acetonitrile) to 100% Solvent B over a period of 30 min at a flow rate 1 ml/min. Retention time 14.07 min. For semi-preparative HPLC separation, column (5 µ, 250 × 10 mm) and flow rate 3ml/min may be used. The appropriate fraction was collected and lyophilized. The same procedure may be used for synthesis of 18FDG or 19FDG.

### Example 16

Determination of blood serum level and immunohistochemical staining of GASP-1 or NXN2 by ELISA.

The serum concentration are determined using an ELISA kit from Proplex Technologies. The kit comprises a coating peptide for coating an ELISA plate, a Standard peptide for standard curve construction, Biotinylated anti-GASP-1 IgG for complex with GASP-1 peptide, Streptavidin-HRP for binding to biotinylated IgG (commercially available from Thermo Fisher Scientific), TMB ultra color reagent for detecting HRP activity in ELISA (commercially available from Thermo Fisher Scientific). Other solutions used include 1% Bovine serum albumin (BSA) in Phosphate buffered saline (PBS), sterile, Tris-buffered saline with 0.05%Tween-20 (TBST), 1N Sulfuric acid (as stop solution for the color reaction). ELISA Plates (microplate, 96 Well, PS, ELISA, Microlon 600, high protein binding, F-bottom, crystal clear) are commercially available e.g. from Microlon.

The method may be similarly performed using for determination of NXN2 using anti-NXN2 IgG. Antibodies to GASP-1 (e.g unique 16 amino acid peptide sequence EEASPEAVAGVGFESK) or to NXN-2 may be made by the injection of selected peptides of the core protein in rabbits, collecting and purifying the sera, e.g. by affinity column for obtaining the IgG fraction, which can used for ELISA as described above.

The ELISA plate (96 wells) is coated with 500 pg of Coating Peptide, incubated overnight at room temperature with shaking (or alternatively, for 2 hours at room temperature). Blocking of unoccupied sites is performed with BSA, then washing is performed with TBST. The ELISA is carried out in duplicate or triplicate. A serum sample is diluted 1:20 with TBST. 50 µl of diluted serum sample (or standard solution) is added to the wells of the coated plate. Then 50 µl of diluted biotinylated antibody is added sequentially to each well. A timer is started when diluted biotinylated antibody is added to the well containing no peptide (control well). The antibody is incubated with shaking for one hour at room temperature. Then the solution is aspirated and the plate is washed three times with TBS-Tween buffer. The buffer is aspirated and 100 µl diluted Streptavidin-HRP is added sequentially to the wells, and incubated with shaking for 1 hour. Then the solution is aspirated and the plate is washed three times with TBS-Tween buffer. The solution is aspirated and 100 µl TMB ultra solution is added sequentially, and incubated with shaking for about 30 min. 50 µl 1 N sulfuric acid stop solution are added to each well. Absorbance of wells is read at 450 nm. If serum samples contain too much peptides/antigen, they may give a low reading and may have to be diluted more than 1 to 20 (e.g. 1 to 40). Results are analyzed with software to plot a standard curve and unknowns are interpolated from the generated standard curve, e.g. using the one site total equation from GraphPad, La Jolla, California. Immunohistochemical staining of cells and tissues may be performed by standard methods, e.g. as described in Wu, et al., Cancer Cell 2017, Vol. 31, issue 3, p368-382.

### Reference Example 17

DZ-SIM amide may be synthesized as illustrated in the reaction scheme below, e.g. as follows: To a solution of simvastatin (1 g, 2.39 mmol, 1 eq.) in acetonitrile, propane-1,3-diamine (1 mL, 11.95 mmol, 5 eq.) was added. The mixture was refluxed with continuous stirring for 4 h. The solvent was removed under reduced pressure and the product was dried in high vacuum. The resulting product 5 was used without further purification. The mixture of DZ 1 (500 mg, 0.71 mmol) 1-ethyl-3-(3-dimethyllaminopropyl) carbodiie hydrochloride (204 mg, 1.07 mmol) and 1-hydroxy-7-azabenzotriazole (115 mg, 0.85 mmol) were dissolved in 10.0 mL CH2Cl2 solution. The mixture was stirred for 15 min, then compound 5 (350 mg, 0.71 mmol) was added and stirred for additional 2 hours at rt. The solvent was removed under reduced pressure and the product was purified by C18-RP silica chromatography elution with methanol-water to afford desired product 6 as a dark green solid 327 mg (39%). Mass spectrum (ESI) 1179.65 [M+H]+.

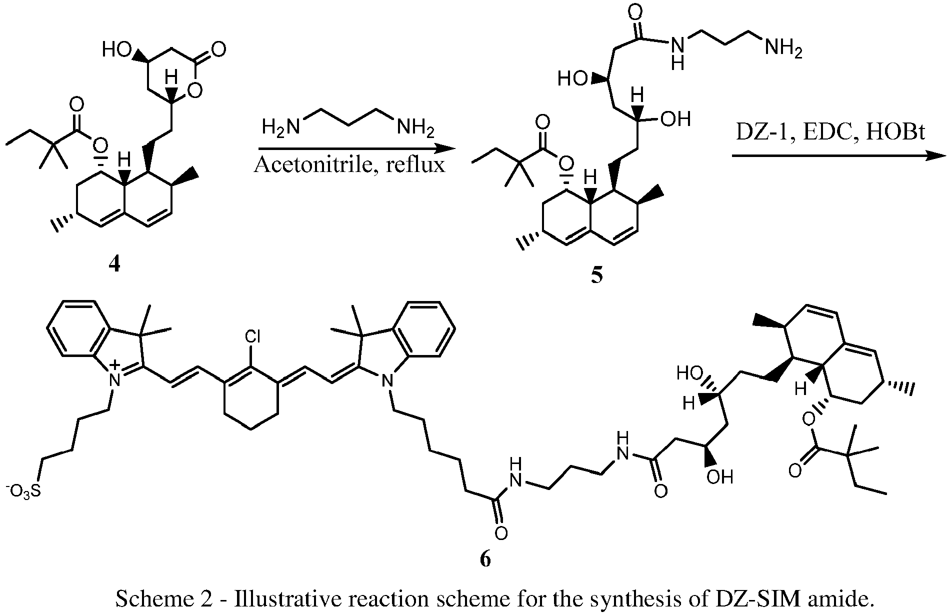

## Claims

1. A dye-therapeutic moiety conjugate having the following structure:

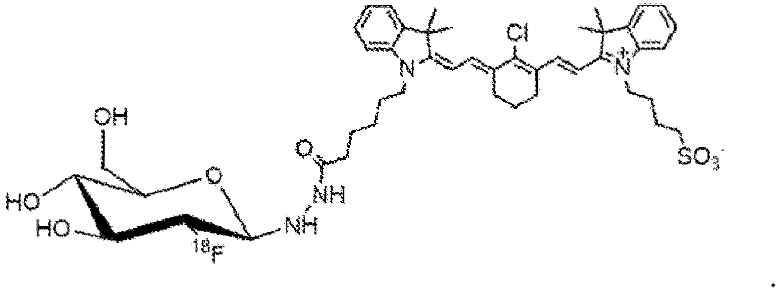

2. The dye-therapeutic moiety conjugate of claim 1 for use in a method of identifying, imaging or localizing cancerous cells or tumors in a patient in need thereof, the method comprising:
providing the dye-therapeutic moiety conjugate of claim 1;
administering the dye-therapeutic moiety conjugate to the patient; and
imaging the location of the cancerous cells or tumors.

## Patentansprüche

1. Konjugat aus Farbstoff und therapeutischer Einheit mit der folgenden Struktur:

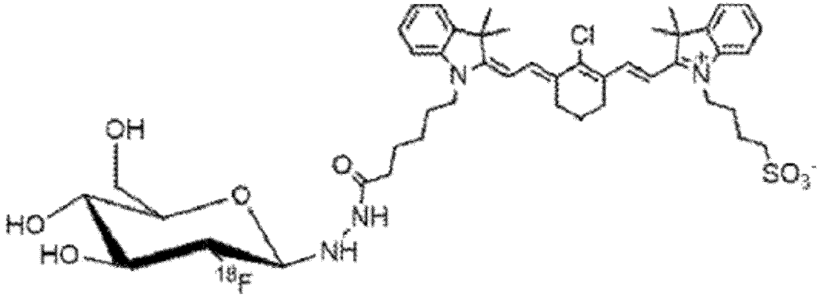

2. Konjugat aus Farbstoff und therapeutischer Einheit nach Anspruch 1 zur Verwendung in einem Verfahren zur Identifizierung, Abbildung oder Lokalisierung von Krebszellen oder Tumoren in einem Patienten, der dieser bedarf, wobei das Verfahren umfasst:
Bereitstellen des Konjugats aus Farbstoff und therapeutischer Einheit nach Anspruch 1;
Verabreichen des Konjugats aus Farbstoff und therapeutischer Einheit an den Patienten und
Abbilden der Stelle der Krebszellen oder Tumoren.

## Revendications

1. Conjugué de colorant-fragment thérapeutique ayant la structure suivante :

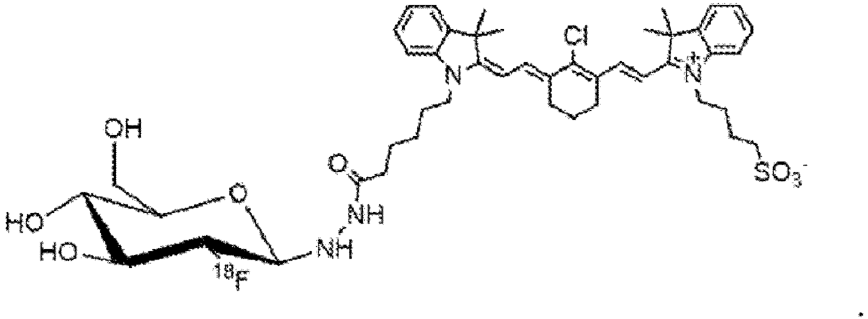

2. Conjugué de colorant-fragment thérapeutique selon la revendication 1 destiné à être utilisé dans un procédé d'identification, d'imagerie ou de localisation de cellules cancéreuses ou tumeurs chez un patient en ayant besoin, le procédé comprenant :
fournir le conjugué de colorant-fragment thérapeutique selon la revendication 1 ;
administrer le conjugué de colorant-fragment thérapeutique au patient ; et
imager l'emplacement des cellules cancéreuses ou tumeurs.
